# EUROPEAN PATENT SPECIFICATION

(11) **EP 0 570 196 B1**
(45) Date of publication and mention of the grant of the patent: **06.03.1996**
(21) Application number: 93303637.8
(22) Date of filing: 11.05.1993
(51) Int. Cl.: C07D 309/10, A61K 31/35, C07D 407/12, C07D 405/12, C07D 309/28

(54) **Oxime derivatives**
Oximderivate
Dérivés d'oximes

(30) Priority: 12.05.1992 EP 92401291
(43) Date of publication of application: 18.11.1993
(73) Proprietor: ZENECA LIMITED, London W1Y 6LN (GB); ZENECA Pharma S.A., F-95022 Cergy Pontoise Cédex (FR)
(72) Inventor: Edwards, Philip Neil, Bramhall, Cheshire SK7 2NW (GB); Large, Michael Stewart, Stoke-on-Trent, Staffordshire ST8 6QU (GB)
(74) Representative: Tait, Brian Steele

(56) References cited:
- EP-A- 0 375 404
- EP-A- 0 385 662

## Description

This invention concerns oxime derivatives and more particularly oxime derivatives which are inhibitors of the enzyme 5-lipoxygenase (hereinafter referred to as 5-LO). The invention also concerns processes for the manufacture of said oxime derivatives and novel pharmaceutical compositions containing them. Also included in the invention is the use of said oxime derivatives in the production of medicaments for use in the treatment of various inflammatory and/or allergic diseases in which the direct or indirect products of 5-LO catalysed oxidation of arachidonic acid are involved.

As stated above the oxime derivatives described hereinafter are inhibitors of 5-LO, which enzyme is known to be involved in catalysing the oxidation of arachidonic acid to give rise via a cascade process to the physiologically active leukotrienes such as leukotriene B₄ (LTB₄) and the peptido-lipid leukotrienes such as leukotriene C₄ (LTC₄) and leukotriene D₄ (LTD₄) and various metabolites.

The biosynthetic relationship and physiological properties of the leukotrienes are summarised by G.W. Taylor and S.R. Clarke in Trends in Pharmacological Sciences, 1986, 7, 100-103. The leukotrienes and their metabolites have been implicated in the production and development of various inflammatory and allergic diseases such as inflammation of the joints (especially rheumatoid arthritis, osteoarthritis and gout), inflammation of the gastrointestinal tract (especially inflammatory bowel disease, ulcerative colitis and gastritis), skin disease (especially psoriasis, eczema and dermatitis) and respiratory disease (especially asthma, bronchitis and allergic rhinitis), and in the production and development of various cardiovascular and cerebrovascular disorders such as myocardial infarction, angina and peripheral vascular disease. In addition the leukotrienes are mediators of inflammatory diseases by virtue of their ability to modulate lymphocyte and leukocyte function. Other physiologically active metabolites of arachidonic acid, such as the prostaglandins and thromboxanes, arise via the action of the enzyme cyclooxygenase on arachidonic acid.

It is disclosed in European Patent Application Nos 0375404 A2 and 0385662 A2 that certain heterocyclic derivatives possess inhibitory properties against 5-LO. Furthermore European Patent Applications Nos. 0409413 and 0420511 are also concerned with heterocyclic derivatives which possess inhibitory properties against 5-LO. We have now discovered that certain oxime derivatives which possess some structural features which are similar to those of the compounds disclosed in the above-mentioned applications but which possess other structural features, in particular oxime groups, which were not envisaged in those earlier applications are effective inhibitors of the enzyme 5-LO and thus of leukotriene biosyntheses. Thus such compounds are of value as therapeutic agents in the treatment of, for example, allergic conditions, psoriasis, asthma, cardiovascular and cerebrovascular disorders, and/or inflammatory and arthritic conditions, mediated alone or in part by one or more leukotrienes.

According to the invention there is provided an oxime derivative of the formula I 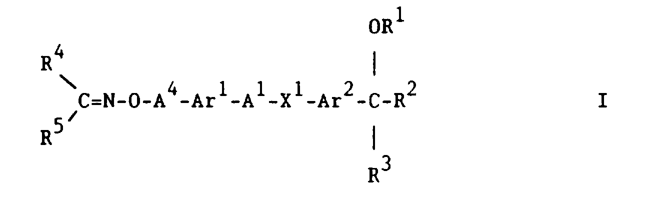
wherein R⁴ is hydrogen, (1-4C)alkyl, halogeno-(2-4C)alkyl, hydroxy-(2-4C)alkyl, cyano-(1-4C)alkyl, phenyl or phenyl-(1-4C)alkyl;
R⁵ is hydrogen, (1-4C)alkyl, halogeno-(2-4C)alkyl, hydroxy-(2-4C)alkyl, cyano-(1-4C)alkyl, phenyl or phenyl-(1-4C)alkyl, or a heteroaryl moiety selected from pyridyl, pyrimidinyl, pyrazinyl, pyridazinyl, furyl, thienyl, oxazolyl and thiazolyl;
or R⁴ and R⁵ together form a group of the formula -A-X-A³- which together with the carbon atom to which A and A³ are attached define a ring having 5 or 6 ring atoms, wherein each of A and A³ is independently (1-3C)alkylene and X is oxy, thio, sulphinyl, sulphonyl or imino, and which ring may optionally bear one or two substituents selected from hydroxy, (1-4C)alkyl and (1-4C)alkoxy;
A⁴ is (1-4C)alkylene which may optionally bear one or two substituents selected from (1-4C)alkyl, phenyl and phenyl-(1-4C)alkyl;
wherein each phenyl or phenyl-(1-4C)alkyl group, or each heteroaryl moiety in R⁴, R⁵ or A⁴ may optionally bear one or two substituents selected from halogeno, cyano, trifluoromethyl, hydroxy, amino, nitro, (1-4C)alkyl, (1-4C)alkoxy, (1-4C)alkylamino, di-(1-4C)alkylamino, (1-4C)alkylthio, (1-4C)alkylsulphinyl and (1-4C)alkylsulphonyl;
Ar¹ is phenylene, pyridinediyl or pyrimidinediyl which may optionally bear one or two substituents selected from halogeno, cyano, trifluoromethyl, hydroxy, amino, (1-4C)alkyl, (1-4C)alkoxy, (1-4C)alkylamino and di-(1-4C)alkylamino;
A¹ is a direct link to X¹, or A¹ is (1-4C)alkylene;
X¹ is oxy, thio, sulphinyl or sulphonyl;
Ar is phenylene, pyridinediyl, pyrimidinediyl, thiophenediyl, furandiyl or thiazolediyl which may optionally bear one or two substituents selected from halogeno, cyano, trifluoromethyl, hydroxy, amino, (1-4C)alkyl, (1-4C)alkoxy, (1-4C)alkylamino and di-(1-4C)alkylamino;
R¹ is hydrogen, (1-4C)alkyl, (3-4C)alkenyl or (3-4C)alkynyl; and
R and R³ together form a group of the formula -A-X-A³- which together with the carbon atom to which A and A³ are attached define a ring having 5 or 6 ring atoms, wherein each of A and A³ is independently (1-3C)alkylene and X is oxy, thio, sulphinyl, sulphonyl or imino, and which ring may optionally bear one or two substituents selected from hydroxy, (1-4C)alkyl and (1-4C)alkoxy;
or wherein R¹ and R together form a group of the formula -A-X-A³ - which together with the oxygen atom to which A is attached and with the carbon atom to which A³ is attached define a ring having 5 or 6 ring atoms, wherein A and A³, which may be the same or different, each is (1-3C)alkylene and X is oxy, thio, sulphinyl or sulphonyl and which ring may bear one, two or three (1-4C)alkyl substituents, and wherein R³ is (1-4C)alkyl, (2-4C)alkenyl or (2-4C)alkynyl;
or a pharmaceutically-acceptable salt thereof.

According to a further feature of the invention there is provided an oxime derivative of the formula I
wherein R⁴ is hydrogen, (1-4C)alkyl, halogeno-(2-4C)alkyl, hydroxy-(2-4C)alkyl, cyano-(1-4C)alkyl, phenyl or phenyl-(1-4C)alkyl;
R⁵ is hydrogen, (1-4C)alkyl, halogeno-(2-4C)alkyl, hydroxy-(2-4C)alkyl, cyano-(1-4C)alkyl, phenyl or phenyl-(1-4C)alkyl;
or R⁴ and R⁵ together form a group of the formula -A-X-A³- which together with the carbon atom to which A and A³ are attached define a ring having 5 or 6 ring atoms, wherein each of A and A³ is independently (1-3C)alkylene and X is oxy, thio, sulphinyl, sulphonyl or imino, and which ring may optionally bear one or two substituents selected from hydroxy, (1-4C)alkyl and (1-4C)alkoxy;
A⁴ is (1-4C)alkylene which may optionally bear one or two substituents selected from (1-4C)alkyl, phenyl and phenyl-(1-4C)alkyl;
wherein each phenyl or phenyl-(1-4C)alkyl group in R⁴, R⁵ or A⁴ may optionally bear one or two substituents selected from halogeno, cyano, trifluoromethyl, hydroxy, amino, (1-4C)alkyl, (1-4C)alkoxy, (1-4C)alkylamino, di-(1-4C)alkylamino, (1-4C)alkylthio, (1-4C)alkylsulphinyl and (1-4C)alkylsulphonyl;
Ar¹ is phenylene, pyridinediyl or pyrimidinediyl which may optionally bear one or two substituents selected from halogeno, cyano, trifluoromethyl, hydroxy, amino, (1-4C)alkyl, (1-4C)alkoxy, (1-4C)alkylamino and di-(1-4C)alkylamino;
A¹ is a direct link to X¹, or A¹ is (1-4C)alkylene;
X¹ is oxy, thio, sulphinyl or sulphonyl;
Ar is phenylene, pyridinediyl, pyrimidinediyl, thiophenediyl, furandiyl or thiazolediyl which may optionally bear one or two substituents selected from halogeno, cyano, trifluoromethyl, hydroxy, amino, (1-4C)alkyl, (1-4C)alkoxy, (1-4C)alkylamino and di-(1-4C)alkylamino;
R¹ is (1-4C)alkyl, (3-4C)alkenyl or (3-4C)alkynyl; and
R and R³ together form a group of the formula -A-X-A³- which together with the carbon atom to which A and A³ are attached define a ring having 5 or 6 ring atoms, wherein each of A and A³ is independently (1-3C)alkylene and X is oxy, thio, sulphinyl, sulphonyl or imino, and which ring may optionally bear one or two substituents selected from hydroxy, (1-4C)alkyl and (1-4C)alkoxy;
or a pharmaceutically-acceptable salt thereof.

The chemical formulae referred to herein by Roman numerals are set out for convenience on a separate sheet hereinafter.

In this specification the generic term "alkyl" includes both straight-chain and branched-chain alkyl groups. However references to individual alkyl groups such as "propyl" are specific for the straight-chain version only and references to individual branched-chain alkyl groups such as "isopropyl" are specific for the branched-chain version only. An analogous convention applies to other generic terms.

It is to be understood that, insofar as certain of the compounds of the formula I defined above may exhibit the phenomenon of tautomerism and any formula drawing presented herein may represent only one of the possible tautomeric forms, the invention includes in its definition any tautomeric form of a compound of the formula I which possesses the property of inhibiting 5-LO and is not to be limited merely to any one tautomeric form utilised within the formulae drawings.

It is further to be understood that, insofar as it is well known that oxime derivatives may exist in different geometric isomeric forms, commonly designated as (E)- or (Z)-isomers, the invention includes in its definition any such geometric isomeric form which possesses the property of inhibiting 5-LO. The separation of such geometric isomeric forms may be possible by the standard laboratory techniques of organic chemistry such as by chromatographic separation of a mixture of said isomeric forms or by crystallisation of one such isomeric form from a mixture thereof.

It is further to be understood that, insofar as certain of the compounds of formula I defined above may exist in optically active or racemic forms by virtue of one or more asymmetric carbon atoms, the invention includes in its definition any such optically active or racemic form which possesses the property of inhibiting 5-LO. The synthesis of optically active forms may be carried out by standard techniques of organic chemistry well known in the art, for example by synthesis from optically active starting materials or by resolution of a racemic form.

Suitable values for the generic terms referred to above include those set out below.

A suitable value for R⁴ or R⁵ when it is (1-4C)alkyl is, for example, methyl, ethyl, propyl, isopropyl or butyl; when it is halogeno-(2-4C)alkyl is, for example, 2-fluoroethyl, 2-chloroethyl, 2-bromoethyl, 3-fluoropropyl or 3-chloropropyl; when it is hydroxy-(2-4C)alkyl is, for example, 2-hydroxyethyl or 3-hydroxypropyl; when it is cyano-(1-4C)alkyl is, for example, cyanomethyl, 1-cyanoethyl, 2-cyanoethyl, 2-cyanoprop-2-yl or 3-cyanopropyl; and when it is phenyl-(1-4C)alkyl is, for example, benzyl, phenethyl or 3-phenylpropyl.

A suitable value for R⁵ when it is a heteroaryl moiety selected from pyridyl, pyrimidinyl, pyrazinyl, pyridazinyl, furyl, thienyl, oxazolyl and thiazolyl is, for example 2-, 3- or 4-pyridyl, 2-, 4- or 5-pyrimidinyl, 2-pyrazinyl, 3- or 4-pyridazinyl, 2- or 3-furyl, 2- or 3-thienyl, 2-, 4- or 5-oxazolyl or 2-, 4- or 5-thiazolyl.

A suitable value for A⁴ when it is (1-4C)alkylene is, for example, methylene, ethylene or trimethylene. Suitable values for the substituents which may be present on A⁴ include, for example:-
- for (1-4C)alkyl:: methyl, ethyl, propyl, isopropyl and butyl;
- for phenyl-(1-4C)alkyl:: benzyl and phenethyl.
When two such substituents are present they may be located on any of the carbon atoms in said (1-4C)alkylene group, including, for example, both substituents on the same carbon atom.

Suitable values for substituents which may be present when R⁴ or R⁵ is phenyl or phenyl-(1-4C)alkyl or a heteroaryl moiety, when A⁴ bears a phenyl or phenyl-(1-4C)alkyl substituent, or for substituents on Ar¹ or Ar, include, for example:-
- for halogeno:: fluoro, chloro and bromo;
- for (1-4C)alkyl:: methyl, ethyl, propyl and isopropyl;
- for (1-4C)alkoxy:: methoxy, ethoxy, propoxy and isopropoxy;
- for (1-4C)alkylamino:: methylamino, ethylamino and propylamino;
- for di-(1-4C)alkylamino:: dimethylamino, diethylamino and N-ethyl-N-methylamino;
- for (1-4C)alkylthio:: methylthio, ethylthio and propylthio;
- for (1-4C)alkylsulphinyl:: methylsulphinyl, ethylsulphinyl and propylsulphinyl;
- for (1-4C)alkylsulphonyl:: methylsulphonyl, ethylsulphonyl and propylsulphonyl.

A suitable value for Ar¹ or Ar when it is phenylene is, for example, 1,3- or 1,4-phenylene.

A suitable value for A¹ when it is (1-4C)alkylene is, for example, methylene, ethylene or trimethylene.

A suitable value for Ar¹ or Ar when it is pyridinediyl, pyrimidinediyl, thiophenediyl, furandiyl or thiazolediyl is, for example, 2,4-, 2,5- or 3,5-pyridinediyl, 4,6-pyrimidinediyl, 2,4- or 2,5-thiophenediyl, 2,4- or 2,5-furandiyl or 2,4-or 2,5-thiazolediyl.

A suitable value for R¹ when it is (1-4C)alkyl is, for example, methyl, ethyl, propyl or butyl; when it (3-4C)alkenyl is, for example allyl, 2-butenyl or 3-butenyl; and when it is (3-4C)alkynyl is, for example, 2-propynyl or 2-butynyl.

When R and R³ together, when R⁴ and R⁵ together, or when R¹ and R together form a group of the formula -A-X-A³- then a suitable value for A or A³, which may be the same or different, when each is (1-3C)alkylene is, for example, methylene, ethylene or trimethylene. Suitable values for the substituents which may be present on the ring so formed include, for example:-
- for (1-4C)alkyl:: methyl, ethyl, propyl, isopropyl, butyl and isobutyl;
- for (1-4C)alkoxy:: methoxy, ethoxy, propoxy, isopropoxy and butoxy.
Said substituents may be located on any available position including, when the substituent is (1-4C)alkyl, on the nitrogen atom when X is imino.

A suitable value for R³ when it is (1-4C)alkyl is, for example, methyl, ethyl, propyl or butyl; when it is (2-4C)alkenyl is, for example, vinyl, allyl, 2-butenyl or 3-butenyl; and when it is (2-4C)alkynyl is, for example, ethynyl, 2-propynyl or 2-butynyl.

A suitable pharmaceutically-acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically-acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Particular compounds of the invention include, for example, oxime derivatives of the formula I, or pharmaceutically-acceptable salts thereof, wherein:-
(a) R⁴ is hydrogen, (1-4C)alkyl, phenyl or phenyl-(1-4C)alkyl and wherein said phenyl or phenyl-(1-4C)alkyl group may optionally bear one or two substituents selected from halogeno, (1-4C)alkyl and (1-4C)alkoxy; and R⁵, A⁴, Ar¹, A¹, X¹, Ar, R¹, R and R³ have any of the meanings defined hereinbefore or in this section defining particular compounds;
(b) R⁵ is hydrogen, (1-4C)alkyl, halogeno-(2-4C)alkyl, cyano-(1-4C)alkyl, phenyl or phenyl-(1-4C)alkyl and wherein said phenyl or phenyl-(1-4C)alkyl group may optionally bear one or two substituents selected from halogeno, (1-4C)alkyl and (1-4C)alkoxy; and R⁴, A⁴, Ar¹, A¹, X¹, Ar, R¹, R and R³ have any of the meanings defined hereinbefore or in this section defining particular compounds;
(c) R⁵ is a heteroaryl moiety selected from pyridyl, pyrimidinyl, furyl, thienyl and thiazolyl which may optionally bear one or two substituents selected from halogeno, nitro, (1-4C)alkyl and (1-4C)alkoxy; and R⁴, A⁴, Ar¹, A¹, X¹, Ar, R¹, R and R³ have any of the meanings defined hereinbefore or in this section defining particular compounds;
(d) A⁴ is methylene or ethylene which may optionally bear one or two substituents selected from methyl, ethyl, phenyl and benzyl; and R⁴, R⁵, Ar¹, A¹, X¹, Ar, R¹, R and R³ have any of the meanings defined hereinbefore or in this section defining particular compounds;
(e) A⁴ is methylene which may optionally bear one or two substituents selected from methyl and ethyl; and R⁴, R⁵, Ar¹, A¹, X¹, Ar, R¹, R and R³ have any of the meanings defined hereinbefore or in this section defining particular compounds;
(f) Ar¹ is phenylene which may optionally bear one or two substituents selected from halogeno, (1-4C)alkyl and (1-4C)alkoxy; and R⁴, R⁵, A⁴, A¹, X¹, Ar, R¹, R and R³ have any of the meanings defined hereinbefore or in this section defining particular compounds;
(g) Ar¹ is 2,4-, 2,5- or 3,5-pyridinediyl; and R⁴, R⁵, A⁴, A¹, X¹, Ar, R¹, R and R³ have any of the meanings defined hereinbefore or in this section defining particular compounds;
(h) Ar¹ is 2,5-pyridinediyl (with the -A¹-X¹- group in the 2-position); and R⁴, R⁵, A⁴, A¹, X¹, Ar, R¹, R and R³ have any of the meanings defined hereinbefore or in this section defining particular compounds;
(i) A¹ is a direct link to X¹; and R⁴, R⁵, A⁴, Ar¹, Ar, X¹, R¹, R and R³ have any of the meanings defined hereinbefore or in this section defining particular compounds;
(j) A¹ is (1-4C)alkylene and X¹ is oxy; and R⁴, R⁵, A⁴, Ar¹, Ar, R¹, R and R³ have any of the meanings defined hereinbefore or in this section defining particular compounds;
(k) A¹ is a direct link to X¹ and X¹ is thio; and R⁴, R⁵, A⁴, Ar¹, Ar, R¹, R and R³ have any of the meanings defined hereinbefore or in this section defining particular compounds;
(l) Ar is phenylene which may optionally bear one or two substituents selected from halogeno, trifluoromethyl, amino, (1-4C)alkyl and (1-4C)alkoxy, or Ar is pyridinediyl, pyrimidinediyl or thiophenediyl; and R⁴, R⁵, A⁴, Ar¹, A¹, X¹, R¹, R and R³ have any of the meanings defined hereinbefore or in this section defining particular compounds;
(m) Ar is thiophenediyl or thiazolediyl; and R⁴, R⁵, A⁴, Ar¹, A¹, X¹, R¹, R and R³ have any of the meanings defined hereinbefore or in this section defining particular compounds;
(n) R¹ is (1-4C)alkyl; and R⁴, R⁵, A⁴, Ar¹, A¹, X¹, Ar, R and R³ have any of the meanings defined hereinbefore or in this section defining particular compounds;
(o) R and R³ together form a group of the formula -A-X-A³- which together with the carbon atom to which A and A³ are attached define a ring having 5 or 6 ring atoms, wherein each of A and A³ is independently (1-3C)alkylene and X is oxy, and which ring may optionally bear one or two (1-4C)alkyl substituents; and R⁴, R⁵, A⁴, Ar¹, A¹, X¹, Ar and R¹ have any of the meanings defined hereinbefore or in this section defining particular compounds; and
(p) R¹ and R together form a group of the formula -A-X-A³- which together with the oxygen atom to which A is attached and with the carbon atom to which A³ is attached define a ring having 5 ring atoms, wherein each of A and A³ is methylene and X is oxy, and which ring may optionally bear one or two substituents selected from methyl and ethyl, and R³ is methyl or ethyl; and R⁴, R⁵, A⁴, Ar¹, A¹, X¹ and Ar have any of the meanings defined hereinbefore or in this section defining particular compounds.

A preferred compound of the invention comprises an oxime derivative of the formula I wherein R⁴ is hydrogen, methyl, ethyl, propyl, phenyl or benzyl, and wherein said phenyl or benzyl group may optionally bear one or two substituents selected from fluoro, chloro, methyl and methoxy;
R⁵ is hydrogen, methyl, ethyl, propyl, phenyl or benzyl, and wherein said phenyl or benzyl group may optionally bear one or two substituents selected from fluoro, chloro, methyl and methoxy;
A⁴ is methylene which may optionally bear one or two substituents selected from methyl, ethyl, phenyl and benzyl, and wherein said phenyl or benzyl group may optionally bear one or two substituents selected from fluoro, chloro, methyl and methoxy;
Ar¹ is 1,4-phenylene which may optionally bear one substituent selected from fluoro, chloro, methyl and methoxy;
A¹ is a direct link to X¹ and X¹ is thio, or A¹ is methylene and X¹ is oxy;
Ar is 1,3- or 1,4-phenylene which may optionally bear one or two substituents selected from fluoro, chloro, trifluoromethyl, amino, methyl and methoxy, or Ar is 3,5-pyridinediyl, 4,6-pyrimidinediyl, 2,4-thiophenediyl or 2,5-thiophenediyl;
R¹ is methyl, ethyl or allyl; and
R and R³ together form a group of the formula -A-X-A³- which together with the carbon atom to which A and A³ are attached define a ring having 6 ring atoms, wherein each of A and A³ is ethylene and X is oxy, and which ring may optionally bear one or two substituents selected from methyl and ethyl;
or a pharmaceutically-acceptable salt thereof.

A further preferred compound of the invention comprises an oxime derivative of the formula I wherein R⁴ is hydrogen, methyl, ethyl, propyl, phenyl or benzyl, and wherein said phenyl or benzyl group may optionally bear one or two substituents selected from fluoro, chloro, methyl and methoxy;
R⁵ is hydrogen, methyl, ethyl, propyl, phenyl, benzyl, pyridyl, pyrimidinyl, furyl, thienyl or thiazolyl, and wherein said 7 last mentioned groups may optionally bear one or two substituents selected from fluoro, chloro, nitro, methyl and methoxy; or R⁴ and R⁵ together form a group of the formula -A-X-A³ wherein A is methylene or ethylene, A³ is ethylene and X is oxy;
A⁴ is methylene which may optionally bear one or two substituents selected from methyl, ethyl, phenyl and benzyl, and wherein said phenyl or benzyl group may optionally bear one or two substituents selected from fluoro, chloro, methyl and methoxy;
Ar¹ is 1,4-phenylene which may optionally bear one substituent selected from fluoro, chloro, methyl and methoxy, or Ar¹ is 2,5-pyridinediyl (with the A¹ group in the 2-position);
A¹ is a direct link to X¹ and X¹ is thio, or A¹ is methylene and X¹ is oxy;
Ar is 1,3- or 1,4-phenylene which may optionally bear one or two substituents selected from fluoro, chloro, trifluoromethyl, amino, methyl and methoxy, or Ar is 3,5-pyridinediyl, 4,6-pyrimidinediyl, 2,4-thiophenediyl, 2,5-thiophenediyl, 2,4-thiazolediyl or 2,5-thiazolediyl;
R¹ is hydrogen, methyl, ethyl or allyl; and
R and R³ together form a group of the formula -A-X-A³ which together with the carbon atom to which A and A³ are attached define a ring having 5 or 6 ring atoms, wherein A is methylene or ethylene, A³ is ethylene and X is oxy, and which ring may optionally bear one or two substituents selected from methyl and ethyl;
or a pharmaceutically-acceptable salt thereof.

A further preferred compound of the invention comprises an oxime derivative of the formula I
wherein R⁴ is hydrogen, methyl, ethyl, phenyl or benzyl;
R⁵ is hydrogen, methyl, ethyl, phenyl or benzyl; A⁴ is methylene which may optionally bear one or two substituents selected from methyl, ethyl, phenyl and benzyl;
wherein each phenyl or benzyl group in R⁴, R⁵ or A⁴ may optionally bear one or two substituents selected from fluoro, chloro, methyl and methoxy;
Ar¹ is 1,4-phenylene;
A¹ is a direct link to X¹;
X¹ is thio;
Ar is 1,3-phenylene which may optionally bear one or two substituents selected from fluoro and chloro, or Ar is 2,4- or 2,5-thiophenediyl;
R¹ is methyl, ethyl or allyl; and
R and R³ together form a group of the formula -A-X-A³- which together with the carbon atom to which A and A³ are attached define a ring having 6 ring atoms, wherein each of A and A³ is ethylene and X is oxy, and which ring may optionally bear one or two methyl substituents;
or a pharmaceutically-acceptable salt thereof.

A further preferred compound of the invention comprises an oxime derivative of the formula I
wherein R⁴ is hydrogen, methyl or phenyl;
R⁵ is methyl, phenyl, 2-pyridyl or 5-nitro-2-furyl;
or R⁴ and R⁵ together form a group of the formula -A-X-A³- wherein each of A and A³ is ethylene and X is oxy;
A⁴ is methylene which may optionally bear a methyl substituent;
Ar¹ is 1,4-phenylene or 2-chloro-1,4-phenylene (with the A¹ group in the 4-position);
A¹ is a direct link to X¹ and X¹ is thio, or A¹ is methylene and X¹ is oxy;
Ar is 1,3-phenylene or 5-fluoro-1,3-phenylene;
R¹ is methyl; and
R and R³ together form a group of the formula -A-X-A³- which together with the carbon atom to which A and A³ are attached define a ring having 6 ring atoms, wherein each of A and A³ is ethylene and X is oxy, and which ring may optionally bear a methyl substituent alpha to X; or a pharmaceutically-acceptable salt thereof.

A further preferred compound of the invention comprises an oxime derivative of the formula I
wherein R⁴ is methyl or phenyl;
R⁵ is methyl or phenyl;
A⁴ is methylene which may optionally bear a methyl substituent;
Ar¹ is 1,4-phenylene;
A¹ is a direct link to X¹;
X¹ is thio;
Ar is 1,3-phenylene or 5-fluoro-1,3-phenylene;
R¹ is methyl; and
R and R³ together form a group of the formula -A-X-A³- which together with the carbon atom to which A and A³ are attached define a ring having 6 ring atoms, wherein each of A and A³ is ethylene and X is oxy, and which ring may optionally bear a methyl substituent alpha to X;
or a pharmaceutically-acceptable salt thereof.

A specific especially preferred compound of the invention is, for example, the following oxime derivative of the formula I, or a pharmaceutically-acceptable salt thereof:- acetone 0-{4-[5-fluoro-3-(4-methoxytetrahydropyran-4-yl)phenylthio]-benzyl}oxime or acetone 0-{4-[5-fluoro-3-(4-methoxytetrahydro-. pyran-4-yl)phenylthio]-α-methylbenzyl}oxime.

A further specific especially preferred compound of the invention is, for example, the following oxime derivative of the formula I, or a pharmaceutically-acceptable salt thereof:-
5-nitrofuran-2-carboxaldehyde 0-{4-[5-fluoro-3-(4-methoxytetrahydropyran-4-yl)phenylthio]benzyl}oxime or acetone 0-{2-chloro-4-[5-fluoro-3-(4-methoxytetrahydropyran-4-yl)phenylthio]benzyl}oxime.

A compound of the invention comprising an oxime derivative of the formula I, or a pharmaceutically-acceptable salt thereof, may be prepared by any process known to be applicable to the preparation of structurally-related compounds. Such procedures are provided as a further feature of the invention and are illustrated by the following representative examples in which, unless otherwise stated, R⁴, R⁵, A⁴, Ar¹, A¹, X¹, Ar, R¹, R and R³ have any of the meanings defined hereinbefore, provided that when there is an amino, imino, alkylamino or hydroxy group in R⁴, R⁵, A⁴, Ar¹, Ar, R¹, R or R³ then any such group may optionally be protected by a conventional protecting group which may be removed when so desired by conventional means.

(a) The reaction, conveniently in the presence of a suitable base, of a compound of the formula II

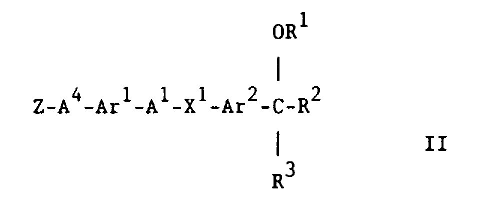

wherein Z is a displaceable group, with an oxime of the formula III

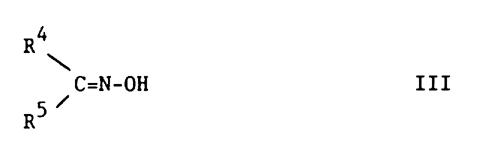

A suitable displaceable group Z is, for example, a halogeno or sulphonyloxy group, for example a chloro, bromo, iodo, methanesulphonyloxy or toluene-4-sulphonyloxy group.

A suitable base for the reaction is, for example, an alkali or alkaline earth metal carbonate, (1-4C)alkoxide, hydroxide or hydride, for example sodium carbonate, potassium carbonate, sodium ethoxide, potassium butoxide, sodium hydroxide, potassium hydroxide, sodium hydride or potassium hydride. The reaction is conveniently performed in a suitable inert solvent or diluent, for example N,N-dimethylformamide, N,N-dimethylacetamide, dimethylsulphoxide, acetone, 1,2-dimethoxyethane or tetrahydrofuran, and at a temperature in the range, for example, 10 to 150°C, conveniently at or near ambient temperature.

The starting materials of the formula II may be obtained by standard procedures of organic chemistry. The preparation of such starting materials is described within the accompanying non-limiting Examples. Alternatively necessary starting materials are obtainable by analogous procedures to those illustrated which are within the ordinary skill of an organic chemist. The disclosures of European Patent Applications Nos. 0375404, 0385662, 0409413 and 0420511 are particularly relevant to the preparation of suitable starting materials.

(b) The reaction, conveniently in the presence of a suitable base or of a suitable acid, of a compound of the formula IV

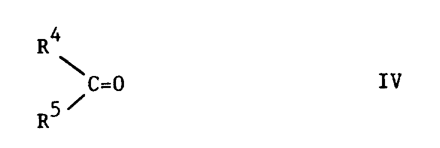

with a hydroxylamine of the formula V

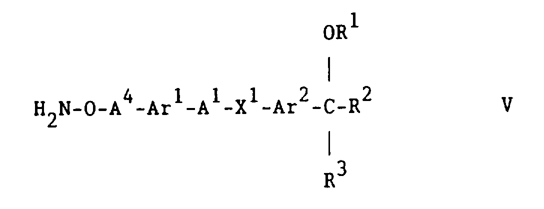

A suitable base for the reaction is, for example, an alkali or alkaline earth metal carbonate, (1-4C)alkoxide, (1-4C)alkanoate, hydroxide or hydride, for example sodium carbonate, potassium carbonate, barium carbonate, sodium ethoxide, potassium butoxide, sodium acetate, sodium hydroxide, potassium hydroxide, sodium hydride or potassium hydride. Alternatively a suitable base for the reaction is, for example, an organic amine base such as pyridine, 2,6-lutidine, collidine, 4-dimethylaminopyridine, triethylamine, N-methylmorpholine or diazabicyclo[5.4.0]undec-7-ene.

A suitable acid for the reaction is, for example, an inorganic acid such as hydrochloric, sulphuric or phosphoric acid, or, for example, an organic acid such as glacial acetic acid, 4-toluenesulphonic acid or trifluoroacetic acid.

The reaction is conveniently performed in a suitable inert solvent or diluent, for example, one or more of water, a (1-4C)alcohol such as methanol, ethanol and propanol, pyridine, 1,2-dimethoxyethane, tetrahydrofuran or a dipolar aprotic solvent such as N,N-dimethylformamide and dimethylsulphoxide. The reaction is conveniently performed at a temperature in the range, for example, 10 to 150°C conveniently at or near 70°C.

The starting materials of the formula V may be obtained by standard procedures of organic chemistry, for example by the reaction of a compound of the formula II with N-hydroxyphthalimide followed by treatment of the phthalimide so produced with hydrazine.

(c) For the production of those compounds of the formula I wherein R¹ and R are linked, the cyclisation, conveniently in the presence of a suitable acid as defined hereinbefore, of a compound of the formula VI

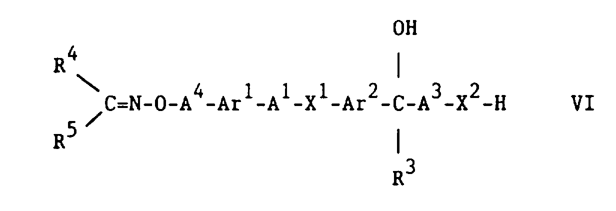

with an appropriate aldehyde or ketone, or with the corresponding hemiacetal or acetal derivative thereof.

The cyclisation reaction is conveniently performed in a suitable inert solvent or diluent, for example 1,2-dimethoxyethane or tetrahydrofuran. Preferably the reaction is performed using the approrpriate aldehyde or ketone as both a reactant and diluent. The cyclisation is effected at a temperature in the range, for example, 20 to 150°C, conveniently at or near the boiling point of the diluent or solvent.

The tertiary alcohol starting material of the formula VI may be obtained by standard procedures of organic chemistry.

The disclosures of European Patent Applications Nos. 0375457, 0385679 and 0409412, together with the procedures within processes (a) and (b) above for the preparation of appropriate oxime groups, are particularly relevant for the preparation of suitable starting materials.

Conventional protecting groups for an amino, imino, alkylamino or hydroxy group which may be present in R⁴, R⁵, A⁴, Ar¹, Ar, R¹, R or R³ are set out hereinafter.

A suitable protecting group for an amino, imino or alkylamino group is, for example, an acyl group for example a (2-4C)alkanoyl group (especially acetyl), a (1-4C)alkoxycarbonyl group (especially methoxycarbonyl, ethoxycarbonyl or tert-butoxycarbonyl), an arylmethoxycarbonyl group (especially benzyloxycarbonyl) or an aroyl group (especially benzoyl). The deprotection conditions for the above protecting groups necessarily vary with the choice of protecting group. Thus, for example, an acyl group such as an alkanoyl or alkoxycarbonyl or an aroyl group may be removed for example, by hydrolysis with a suitable base such as an alkali metal hydroxide, for example lithium or sodium hydroxide. Alternatively an acyl group such as a tert-butoxycarbonyl group may be removed, for example, by treatment with a suitable acid such as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-charcoal.

A suitable protecting group for a hydroxy group is, for example, an acyl group, for example a (2-4C)alkanoyl group (especially acetyl), an aroyl group (especially benzoyl) or an arylmethyl group (especially benzyl). The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-charcoal.

When a pharmaceutically-acceptable salt of a compound of the formula I is required, it may be obtained, for example, by reaction of said compound with a suitable acid or base using a conventional procedure. When an optically active form of a compound of the formula I is required, it may be obtained by carrying out one of the aforesaid procedures using an optically active starting material, or by resolution of a racemic form of said compound using a conventional procedure.

As stated previously, the compounds of the formula I are inhibitors of the enzyme 5-LO. The effects of this inhibition may be demonstrated using one or more of the standard procedures set out below:-
a) An in vitro assay system involving incubating a test compound with heparinised human blood, prior to challenge with the calcium ionophore A23187 and then indirectly measuring the inhibitory effects on 5-LO by assaying the amount of LTB₄ using specific radioimmunoassays described by Carey and Forder (Prostaglandins, Leukotrienes Med., 1986, 22, 57; Prostaglandins, 1984, 28, 666; Brit. J. Pharmacol., 1985, 84, 34P) which involve the use of a protein-LTB₄ conjugate produced using the procedure of Young et alia (Prostaglandins, 1983, 26(4), 605-613). The effects of a test compound on the enzyme cyclooxygenase (which is involved in the alternative metabolic pathway for arachidonic acid and gives rise to prostaglandins, thromboxanes and related metabolites) may be measured at the same time using the specific radioimmunoassay for thromboxane B₂(TxB₂) described by Carey and Forder (see above). This test provides an indication of the effects of a test compound against 5-LO and also cyclooxygenase in the presence of blood cells and proteins. It permits the selectivity of the inhibitory effect on 5-LO or cyclooxygenase to be assessed.
b) An ex vivo assay system, which is a variation of test a) above, involving administration to a group of rats of a test compound (usually orally as the suspension produced when a solution of the test compound in dimethylsulphoxide is added to carboxymethylcellulose), blood collection, heparinisation, challenge with A23187 and radioimmunoassay of LTB₄ and TxB₂. This test provides an indication of the bioavailability of a test compound as an inhibitor of 5-LO or cyclooxygenase.
c) An in vivo system involving measuring the effects of a test compound administered orally against the liberation of LTB₄ induced by zymosan within an air pouch generated within the subcutaneous tissue of the back of male rats. The rats are anaesthetised and air pouches are formed by the injection of sterile air (20ml). A further injection of air (10ml) is similarly given after 3 days. At 6 days after the initial air injection the test compound is administered (usually orally as the suspension produced when a solution of the test compound in dimethylsulphoxide is added to hydroxypropylmethylcellulose), followed by the intrapouch injection of zymosan (lml of a 1% suspension in physiological saline). After 3 hours the rats are killed, the air pouches are lavaged with physiological saline, and the specific radioimmunoassay described above is used to assay LTB₄ in the washings. This test provides an indication of inhibitory effects against 5-LO in an inflammatory milieu.

Although the pharmacological properties of the compounds of the formula I vary with structural changes as expected, in general compounds of the formula I possess 5-LO inhibitory effects at the following concentrations or doses in one or more of the above tests a)-c):-
- Test a):: IC₅₀ (LTB₄) in the range, for example, 0.01-40µM
IC₅₀ (TxB₂) in the range, for example, 40-200µM;
- Test b):: oral ED₅₀(LTB₄) in the range, for example, 0.1-100mg/kg;
- Test c):: oral ED₅₀(LTB₄) in the range, for example, 0.1-50mg/kg.

No overt toxicity or other untoward effects are present in tests b) and/or c) when compounds of the formula I are administered at several multiples of their minimum inhibitory dose or concentration.

Thus, by way of example, the compound acetone 0-{4-[5-fluoro-3-(4-methoxytetrahydropyran-4-yl)phenylthio]benzyl}oxime has an IC₅₀ of 0.15µM against LTB₄ in test a) and an oral ED₅₀ of approximately 1 mg/kg versus LTB₄ in test c); and the compound acetone 0-{4-[5-fluoro-3-(4-methoxytetrahydropyran-4-yl)phenylthio]-α-methylbenzyl}oxime has an IC₅₀ of 0.09µM against LTB₄ in test a) and an oral ED₅₀ of approximately 1.5 mg/kg versus LTB₄ in test c). In general those compounds of the formula I which are particularly preferred have an IC₅₀ of <1µM against LTB₄ in test a) and an oral ED₅₀ of <100 mg/kg against LTB₄ in tests b) and/or c).

These compounds are examples of compounds of the invention which show selective inhibitory properties for 5-LO as opposed to cyclooxygenase, which selective properties are expected to impart improved therapeutic properties, for example, a reduction in or freedom from the gastrointestinal side-effects frequently associated with cyclooxygenase inhibitors such as indomethacin.

According to a further feature of the invention there is provided a pharmaceutical composition which comprises an oxime derivative of the formula I, or a pharmaceutically-acceptable salt thereof, in association with a pharmaceutically-acceptable diluent or carrier.

The composition may be in a form suitable for oral use, for example a tablet, capsule, aqueous or oily solution, suspension or emulsion; for topical use, for example a cream, ointment, gel or aqueous or oily solution or suspension; for nasal use, for example a snuff, nasal spray or nasal drops; for vaginal or rectal use, for example a suppository; for administration by inhalation, for example as a finely divided powder such as a dry powder, a microcrystalline form or a liquid aerosol; for sub-lingual or buccal use, for example a tablet or capsule; or for parenteral use (including intravenous, subcutaneous, intramuscular, intravascular or infusion), for example a sterile aqueous or oily solution or suspension.
In general the above compositions may be prepared in a conventional manner using conventional excipients.

The amount of active ingredient (that is an oxime derivative of the formula I, or a pharmaceutically-acceptable salt thereof) that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient.

According to a further feature of the invention there is provided an oxime derivative of the formula I, or a pharmaceutically-acceptable salt thereof, for use in a method of treatment of the human or animal body by therapy.

The invention also provides the use of an active ingredient as defined above in the production of a medicament for use in a leukotriene mediated disease or medical condition.

The size of the dose for therapeutic or prophylactic purposes of a compound of the formula I will naturally vary according to the nature and severity of the conditions, the age and sex of the animal or patient and the route of administration, according to well known principles of medicine. As mentioned above, compounds of the formula I are useful in treating those allergic and inflammatory conditions which are due alone or in part to the effects of the metabolites of arachidonic acid arising by the linear (5-LO catalysed) pathway and in particular the leukotrienes, the production of which is mediated by 5-LO. As previously mentioned, such conditions include, for example, asthmatic conditions, allergic reactions, allergic rhinitis, allergic shock, psoriasis, atopic dermatitis, cardiovascular and cerebrovascular disorders of an inflammatory nature, arthritic and inflammatory joint disease, and inflammatory bowel diseases.

In using a compound of the formula I for therapeutic or prophylactic purposes it will generally be administered so that a daily dose in the range, for example, 0.5 mg to 75 mg per kg body weight is received, given if required in divided doses. In general lower doses will be administered when a parenteral route is employed. Thus, for example, for intravenous administration, a dose in the range, for example, 0.5 mg to 30 mg per kg body weight will generally be used. Similarly, for administration by inhalation, a dose in the range, for example, 0.5 mg to 25 mg per kg body weight will be used.

Although the compounds of the formula I are primarily of value as therapeutic agents for use in warm-blooded animals (including man), they are also useful whenever it is required to inhibit the enzyme 5-LO. Thus, they are useful as pharmacological standards for use in the development of new biological tests and in the search for new pharmacological agents.

By virtue of their effects on leukotriene production, the compounds of the formula I have certain cytoprotective effects, for example they are useful in reducing or suppressing certain of the adverse gastrointestinal effects of the cyclooxygenase inhibitory non-steroidal anti-inflammatory agents (NSAIA), such as indomethacin, acetylsalicylic acid, ibuprofen, sulindac, tolmetin and piroxicam. Furthermore, co-administration of a 5-LO inhibitor of the formula I with a NSAIA can result in a reduction in the quantity of the latter agent needed to produce a therapeutic effect, thereby reducing the likelihood of adverse side-effects. According to a further feature of the invention there is provided a pharmaceutical composition which comprises an oxime derivative of the formula I, or a pharmaceutically-acceptable salt thereof as defined hereinbefore, in conjunction or admixture with a cyclooxygenase inhibitory non-steroidal anti-inflammatory agent (such as those mentioned above), and a pharmaceutically-acceptable diluent or carrier.

The cytoprotective effects of the compounds of the formula I may be demonstrated, for example in a standard laboratory model which assesses protection against indomethacin-induced or ethanol-induced ulceration in the gastrointestinal tract of rats.

The compositions of the invention may in addition contain one or more therapeutic or prophylactic agents known to be of value for the disease under treatment. Thus, for example a known platelet aggregation inhibitor, hypolipidemic agent, anti-hypertensive agent, beta-adrenergic blocker or a vasodilator may usefully also be present in a pharmaceutical composition of the invention for use in treating a heart or vascular disease or condition. Similarly, by way of example, an anti-histamine, steroid (such as beclomethasone dipropionate), sodium cromoglycate, phosphodiesterase inhibitor or a beta-adrenergic stimulant may usefully also be present in a pharmaceutical composition of the invention for use in treating a pulmonary disease or condition.

The invention will now be illustrated in the following non-limiting Examples in which, unless otherwise stated:-
(i) evaporations were carried out by rotary evaporation in vacuo and work-up procedures were carried out after removal of residual solids by filtration;
(ii) operations were carried out at room temperature, that is in the range 18-25°C and under an atmosphere of an inert gas such as argon;
(iii) column chromatography (by the flash procedure) and medium pressure liquid chromatography (MPLC) were performed on Merck Kieselgel silica (Art. 9385) or Merck Lichroprep RP-18 (Art. 9303) reversed-phase silica obtained from E. Merck, Darmstadt, Germany;
(iv) yields are given for illustration only and are not necessarily the maximum attainable;
(v) the structures of the end-products of the formula I were confirmed by NMR and mass spectral techniques; unless otherwise stated, CDCl₃ solutions of the end-products of the formula I were used for the determination of the NMR spectral data, chemical shift values were measured on the delta scale and the following abbreviations are used: s, singlet; d, doublet; t, triplet; q, quartet; m, multiplet;
(vi) intermediates were not generally fully characterised and purity was assessed by thin layer chromatographic, infra-red (IR) or NMR analysis;
(vii) melting points are uncorrected and were determined using a Mettler SP62 automatic melting point apparatus or an oil-bath apparatus; melting points for the end-products of the formula I were determined after recrystallisation from a conventional organic solvent such as ethanol, methanol, acetone, ether or hexane, alone or in admixture;
(viii) the following abbreviations have been used:-
   - THF: tetrahydrofuran;
   - DMF: N,N-dimethylformamide;
   - NMP: N-methylpyrrolidin-2-one.

### Example 1

Sodium hydride (60% w/w dispersion in mineral oil, 0.02 g) was added to a stirred mixture of acetone oxime (0.036 g), 4-[3-(4-chloromethylphenylthio)-5-fluorophenyl]-4-methoxytetrahydropyran (0.08 g) and DMF (2 ml) and the mixture was stirred at ambient temperature for 2 hours. Acetic acid (0.2 ml) and water (20 ml) were added and the mixture was extracted with diethyl ether. The organic phase was washed with brine, dried (Na₂SO₄) and evaporated. The residue was purified by column chromatography using increasingly polar mixtures of hexane and ethyl acetate as eluent. There was thus obtained acetone 0-{4-[5-fluoro-3-(4-methoxytetrahydropyran-4-yl)phenylthio]benzyl}oxime as an oil (0.06 g, 68%).
NMR Spectrum: 1.88-1.95 (m, 10H), 2.97 (s, 3H), 3.80 (m, 4H), 5.07 (s, 2H), 6.80 (m, 1H), 6.90 (m, 1H), 7.10 (m, 1H), 7.37 (m, 4H).

The 4-[3-(4-chloromethylphenylthio)-5-fluorophenyl]-4-methoxytetrahydropyran used as a starting material was obtained as follows:-

A mixture of 4-(5-fluoro-3-mercaptophenyl)-4-methoxytetrahydropyran (European Patent Application No. 0420511, Example 4 thereof; 2.42 g), potassium hydroxide (0.56 g) and DMF (25 ml) was stirred and heated to 140°C until a clear solution was obtained. 4-Bromobenzaldehyde (2.78 g) and cuprous oxide (0.715 g) were added and the mixture was stirred and heated to 140°C for 90 minutes. The mixture was cooled to ambient temperature and partitioned between diethyl ether and water. The organic phase was washed with brine, dried (MgSO₄) and evaporated. The residue was purified by column chromatography using a 4:1 mixture of petroleum ether (b.p. 40-60°C) and ethyl acetate as eluent. There was thus obtained 4-[5-fluoro-3-(4-methoxytetrahydropyran-4-yl)phenylthio]benzaldehyde (2.38 g, 68%), m.p. 93°C.

Sodium borohydride (0.1 g) was added to a stirred mixture of a portion (0.87 g) of the benzaldehyde so obtained and ethanol (20 ml). The mixture was stirred at ambient temperature for 30 minutes.. Acetic acid (1 ml) and water (50 ml) were added and mixture was extracted with diethyl ether. The organic phase was washed with brine, dried (Na₂SO₄) and evaporated. The residue was purified by column chromatography using increasingly polar mixtures of hexane and ethyl acetate as eluent. There was thus obtained 4-[5-fluoro-3-(4-hydroxymethylphenylthio)phenyl]-4-methoxytetrahydropyran as a gum (0.85 g, 97%).
NMR Spectrum: 1.91 (m, 4H), 2.98 (s, 3H), 3.80 (m, 4H), 4.72 (s, 2H), 6.79 (m, 1H), 6.92 (m, 1H), 7.10 (m, 1H), 7.39 (m, 4H).

Thionyl chloride (0.1 ml) was added to a stirred mixture of a portion (0.075 g) of the material so obtained and methylene chloride (5 ml) and the mixture was stirred at ambient temperature for 30 minutes. The mixture was evaporated, toluene (5 ml) was added and the mixture was evaporated. There was thus obtained 4-[3-(4-chloromethylphenylthio)-5-fluorophenyl]-4-methoxytetrahydropyran as a gum (0.08 g) which was used without further purification.

### Example 2

Using an analogous procedure to that described in Example 1 except that the reactants were heated to 60°C for 2 hours, acetone oxime was reacted with 4-{3-[4-(1-chloroethyl)phenylthio]-5-fluorophenyl}-4-methoxytetrahydropyran to give acetone 0-{4-[5-fluoro-3-(4-methoxytetrahydropyran-4-yl)phenylthio)-α-methylbenzyl}oxime as an oil in 58% yield.
NMR Spectrum: 1.51 (d, 3H), 1.84 (s, 3H), 1.89 (m, 4H), 1.92 (s, 3H), 2.97 (s, 3H), 3.81 (m, 4H), 5.18 (q, 1H), 6.8 (m, 1H), 6.9 (m, 1H), 7.09 (m, 1H), 7.46 (m, 4H).

The 4-{3-[4-(1-chloroethyl)phenylthio]-5-fluorophenyl}-4-methoxytetrahydropyran used as a starting material was obtained as follows:-

Methylmagnesium bromide (3M in THF; 2 ml) was added dropwise to a stirred solution of 4-[5-fluoro-3-(4-methoxytetrahydropyran-4-yl)-phenylthio]benzaldehyde (1.73 g) in THF (20 ml). The mixture was stirred at ambient temperature for 30 minutes. Acetic acid (1 ml) and water (30 ml) were added and the mixture was extracted with diethyl ether. The organic phase was washed with brine, dried (Na₂SO₄) and evaporated. The residue was purified by column chromatography using increasingly polar mixtures of hexane and ethyl acetate as eluent. There was thus obtained 4-{5-fluoro-3-[4-(1-hydroxyethyl)phenylthio]-phenyl}-4-methoxytetrahydropyran as an oil (1.7 g, 94%).
NMR Spectrum: 1.51 (d, 3H), 1.92 (m, 4H), 2.97 (s, 3H), 3.80 (m, 4H), 4.92 (q, 1H), 6.79 (m, 1H), 6.91 (m, 1H), 7.10 (m, 1H), 7.39 (m, 4H).

A mixture of a portion (0.36 g) of the material so obtained, pyridine (0.2 g) and methylene chloride (5 ml) was added dropwise to a stirred solution of thionyl chloride (0.2 ml) in methylene chloride (5 ml) which had been cooled to 0°C. The mixture was stirred at ambient temperature for 1 hour. The mixture was evaporated and the residue was partitioned between diethyl ether and water. The organic phase was dried (Na₂SO₄) and evaporated. There was thus obtained 4-{3-[4-(1-chloroethyl)phenylthio]-5-fluorophenyl}-4-methoxytetrahydropyran as an oil (0.38 g, 10%).
NMR Spectrum 1.85 (d, 3H), 1.90 (m, 4H), 2.98 (s, 3H), 3.80 (m, 4H), 5.08 (q, 1H), 6.87 (m, 1H), 6.96 (m, 1H), 7.14 (m, 1H), 7.38 (m, 4H).

### Example 3

Using an analogous procedure to that described in Example 1, benzophenone oxime was reacted with 4-[3-(4-chloromethylphenylthio)-5-fluorophenyl]-4-methoxytetrahydropyran to give benzophenone 0-{4-[5-fluoro-3-(4-methoxytetrahydropyran-4-yl)phenylthio]benzyl}oxime as an oil in 44% yield.
NMR Spectrum: 1.9 (m, 4H), 2.95 (s, 3H), 3.8 (m, 4H), 5.24 (s, 2H), 6.82 (m, 1H), 6.92 (m, 1H), 7.1 (m, 1H), 7.3-7.5 (m, 14H).

### Example 4

Using an analogous procedure to that described in Example 1 except that the reactants were heated to 60°C for 2 hours, benzophenone oxime was reacted with 4-{3-[4-(1-chloroethyl)phenylthio]-5-fluorophenyl}-4-methoxytetrahydropyran to give benzophenone 0-{4-[5-fluoro-3-(4-methoxytetrahydropyran-4-yl)phenylthio]-α-methylbenzyl}oxime as an oil in 38% yield.
NMR Spectrum: 1.55 (d, 3H), 1.9 (m, 4H), 2.95 (s, 3H), 3.78 (m, 4H), 5.4 (q, 1H), 6.8 (m, 1H), 6.9 (m, 1H), 7.09 (m, 1H), 7.3-7.5 (m, 14H).

### Example 5

Using an analogous procedure to that described in Example 1, tetrahydropyran-4-one oxime was reacted with 4-{3-[4-(1-chloroethyl)phenylthio]-5-fluorophenyl}-4-methoxytetrahydropyran to give tetrahydropyran-4-one 0-{4-[5-fluoro-3-(4-methoxytetrahydropyran-4-yl)phenylthio]-α-methylbenzyl}oxime as an oil in 74% yield.
NMR Spectrum: 1.52 (d, 3H), 1.89 (m, 4H), 2.33 (m, 2H), 2.69 (m, 2H), 2.97 (s, 3H), 3.77 (m, 8H), 5.20 (q, 1H), 6.79 (m, 1H), 6.91 (m, 1H), 7.11 (m, 1H), 7.34 (m, 4H).

The tetrahydropyran-4-one oxime used as a starting material was obtained as follows:-

A mixture of tetrahydropyran-4-one (1 g), hydroxylamine hydrochloride (1 g), sodium acetate (2 g) and ethanol (10 ml) was stirred and heated to reflux for 1 hour. The mixture was evaporated and the residue was partitioned between diethyl ether and water. The organic phase was dried (Na₂SO₄) and evaporated. The residue was recrystallised from a mixture of hexane and ethyl acetate. There was thus obtained tetrahydropyran-4-one oxime (0.75 g, 65%), m.p. 86-87°C.

### Example 6

A mixture of pyridine-2-carboxaldehyde (0.05 g), 0-{4-[5-fluoro-3-(4-methoxytetrahydropyran-4-yl)phenylthio]benzyl}hydroxylamine (0.09 g), glacial acetic acid (0.02 g) and ethanol (2 ml) was stirred and heated to reflux for 1 hour. The mixture was cooled to ambient temperature and partitioned between ethyl acetate and water. The organic phase was washed with water, dried (Na₂SO₄) and evaporated. The residue was purified by column chromatography using increasingly polar mixtures of hexane and ethyl acetate as eluent. There was thus obtained (E)-pyridine-2-carboxaldehyde 0-{4-[5-fluoro-3-(4-methoxytetrahydropyran-4-yl)phenylthio]benzyl}oxime as a gum (0.08 g, 75%).
NMR Spectrum: 1.75-2.05 (m, 4H), 2.96 (s, 3H), 3.75-3.90 (m, 4H), 5.26 (s, 2H), 6.83 (m, 1H), 6.92 (m, 1H), 7.12 (m, 1H), 7.25 (m, 1H), 7.40 (s, 4H), 7.6-7.85 (m, 2H), 8.24 (s, 1H), 8.62 (m, 1H).

The 0-{4-[5-fluoro-3-(4-methoxytetrahydropyran-4-yl)phenylthio]benzyl}hydroxylamine used as a starting material was obtained as follows:-

A mixture of 4-[3-(4-chloromethylphenylthio)-5-fluorophenyl]-4-methoxytetrahydropyran (0.73 g), N-hydroxyphthalimide (0.41 g), potassium carbonate (0.31 g) and DMF (2 ml) was stirred at ambient temperature for 18 hours. The mixture was acidified to pH5 by the addition of glacial acetic acid and partitioned between diethyl ether and water. The organic phase was washed with water and with brine, dried (Na₂SO₄) and evaporated. The residue was purified by column chromatography using increasingly polar mixtures of hexane and ethyl acetate as eluent. There was thus obtained N-{4-[5-fluoro-3-(4-methoxytetrahydropyran-4-yl)phenylthio]benzyloxy}phthalimide as a gum (0.4 g, 41%).

A mixture of a portion (0.35 g) of the phthalimide so obtained, hydrazine hydrate (0.2 ml) and methanol (10 ml) was stirred and heated to reflux for 1 hour. The mixture was cooled to ambient temperature, filtered and evaporated. The residue was purified by column chromatography using increasingly polar mixtures of hexane and ethyl acetate as eluent. There was thus obtained the required starting material as a gum (0.23 g, 89%).

### Example 7

Using an analogous procedure to that described in Example 6, 5-nitrofuran-2-carboxaldehyde was reacted with 0-{4-[5-fluoro-3-(4-methoxytetrahydropyran-4-yl)phenylthio]benzyl}hydroxylamine to give 5-nitrofuran-2-carboxaldehyde 0-{4-[5-fluoro-3-(4-methoxytetrahydropyran-4-yl)phenylthio]benzyl}oxime as a gum in 58% yield.
NMR Spectrum 1.8-2.05 (m, 4H), 2.97 (s, 3H), 3.75-3.95 (m, 4H), 5.25 and 5.32 (2 s's, 2H), 6.33 and 7.33 (2 d's, 1H), 6.8-7.0 (m, 2H), 7.13 (m, 1H), 7.3-7.5 (m, 5H), 7.53 and 8.04 (2 s's, 1H).

### Example 8

Using an analogous procedure to that described in Example 1, acetone oxime was reacted with 4-[3-(4-chloromethylbenzyloxy)-5-fluorophenyl]-4-methoxytetrahydropyran to give acetone 0-{4-[5-fluoro-3-(4-methoxytetrahydropyran-4-yl)phenoxymethyl]benzyl}oxime as a gum in 72% yield.
NMR Spectrum 1.8-2.05 (m, 10H), 2.97 (s, 3H), 3.55-3.70 (m, 4H), 5.04 (s, 2H), 5.07 (s, 2H), 6.61 (m, 1H), 6.70 (m, 1H), 6.80 (m, 1H), 7.39 (t, 4H).

The 4-[3-(4-chloromethylbenzyloxy)-5-fluorophenyl]-4-methoxytetrahydropyran used as a starting material was obtained as follows:-

A mixture of 1,4-benzenedimethanol (4.14 g), sodium hydride (60% w/w dispersion in mineral oil, 0.6 g) and NMP (25 ml) was stirred at ambient temperature for 1 hour. 4-(3,5-Difluorophenyl)-4-methoxytetrahydropyran (European Patent Application No. 0462813, Example 5 thereof; 3.42 g) was added and the mixture was stirred at ambient temperature for 18 hours and then heated to 60°C for 6 hours. The mixture was cooled to ambient temperature and acidified by the addition of glacial acetic acid (5 ml). The mixture was partitioned between diethyl ether and water. The organic phase was washed with water, dried (Na₂SO₄) and evaporated to dryness. The residue was purified by column chromatography using increasingly polar mixtures of hexane and ethyl acetate as eluent. There was thus obtained 4-[5-fluoro-3-(4-hydroxymethylbenzyloxy)phenyl]-4-methoxytetrahydropyran as a solid in 67% yield.

The alcohol so obtained was chlorinated using an analogous procedure to that described in the last paragraph of the portion of Example 1 which is concerned with the preparation of starting materials. There was thus obtained 4-[3-(4-chloromethylbenzyloxy)-5-fluorophenyl]-4-methoxytetrahydropyran as a gum which was used without further purification.

### Example 9

Using an analogous procedure to that described in Example 1, acetone oxime was reacted with 4-[3-(3-chloro-4-chloromethylphenylthio)-5-fluorophenyl]-4-methoxytetrahydropyran to give acetone 0-{2-chloro-4-[5-fluoro-3-(4-methoxytetrahydropyran-4-yl)phenylthio]-benzy})oxime as a gum in 50% yield.
NMR Spectrum 1.8-2.05 (m, 10H), 2.98 (s, 3H), 3.75-3.90 (m, 4H), 5.15 (s, 2H), 6.88 (m, 1H), 6.97 (m, 1H), 7.15 (m, 1H), 7.25 (m, 1H), 7.37 (m, 2H).

The 4-[3-(3-chloro-4-chloromethylphenylthio)-5-fluorophenyl]-4-methoxytetrahydropyran used as a starting material was obtained as follows:-

A mixture of 2-chloro-4-fluorobenzaldehyde (0.526 g), 4-(5-fluoro-3-mercaptophenyl)-4-methoxytetrahydropyran (0.726 g), potassium carbonate (0.46 g) and DMF (5 ml) was stirred and heated to 120°C for 30 minutes. The mixture was cooled to ambient temperature and partitioned between diethyl ether and water. The organic phase was washed with brine, dried (Na₂SO₄) and evaporated. The residue was purified by column chromatography using increasingly polar mixtures of hexane and ethyl acetate as eluent. There was thus obtained 2-chloro-4-[5-fluoro-3-(4-methoxytetrahydropyran-4-yl)phenylthio]benzaldehyde as a gum (1.1 g, 96%).
NMR Spectrum 1.8-2.0 (m, 4H), 3.01 (s, 3H), 3.8-3.9 (m, 4H), 7.1-7.2 (m, 4H), 7.33 (m, 1H), 7.81 (d, 1H), 10.37 (s, 1H).

Using analogous procedures to those described in the second and third paragraphs of the portion of Example 1 which is concerned with the preparation of starting materials, the benzaldehyde so obtained was converted into 4-[3-(3-chloro-4-chloromethylphenylthio)-5-fluorophenyl]-4-methoxytetrahydropyran.

### Example 10

The following illustrate representative pharmaceutical dosage forms containing the compound of formula I, or a pharmaceutically-acceptable salt thereof (hereafter compound X), for therapeutic or prophylactic use in humans:

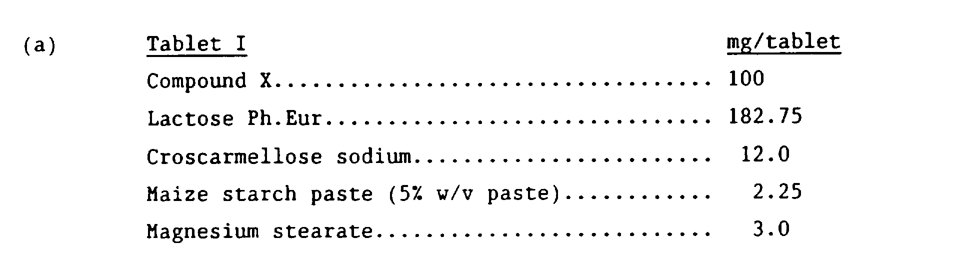

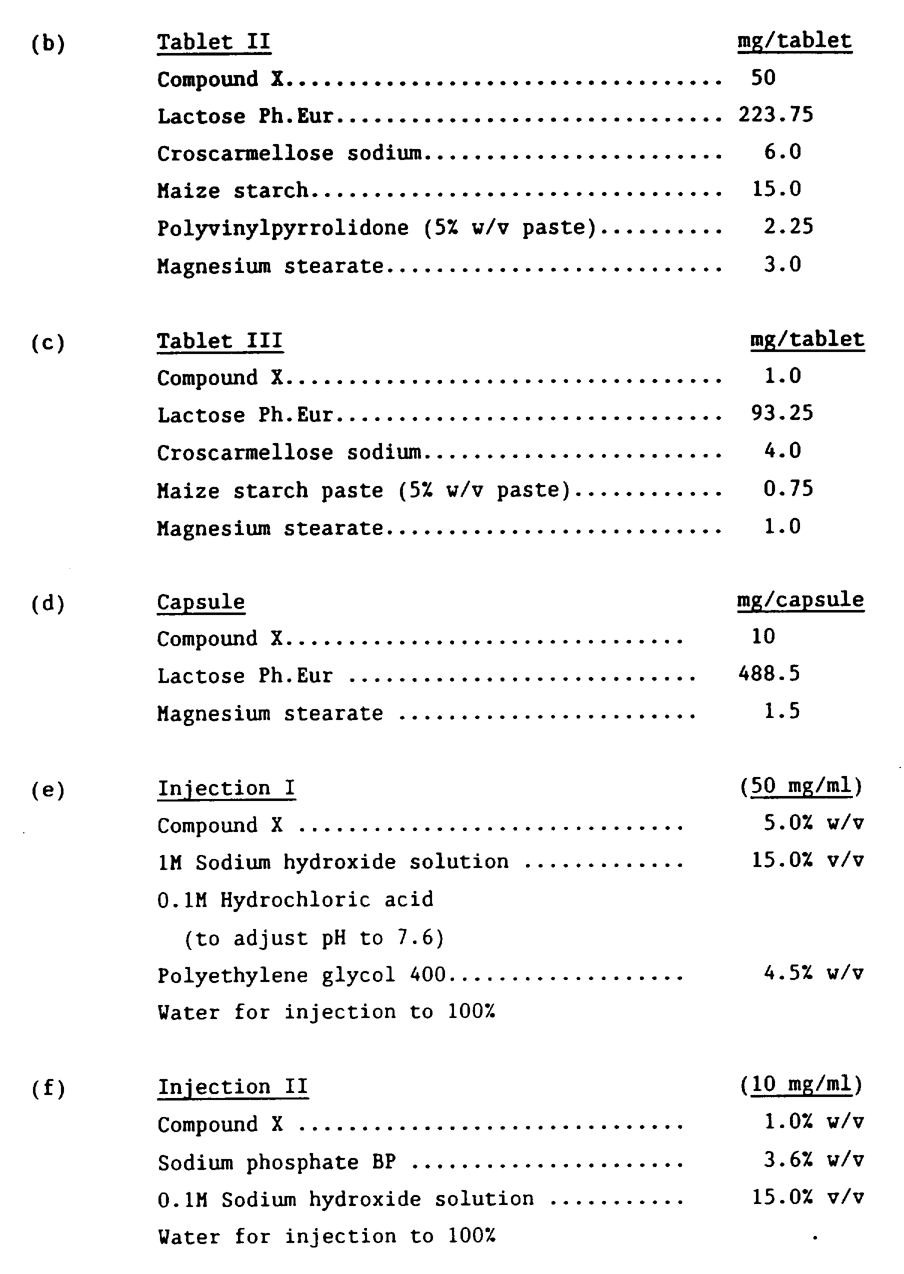

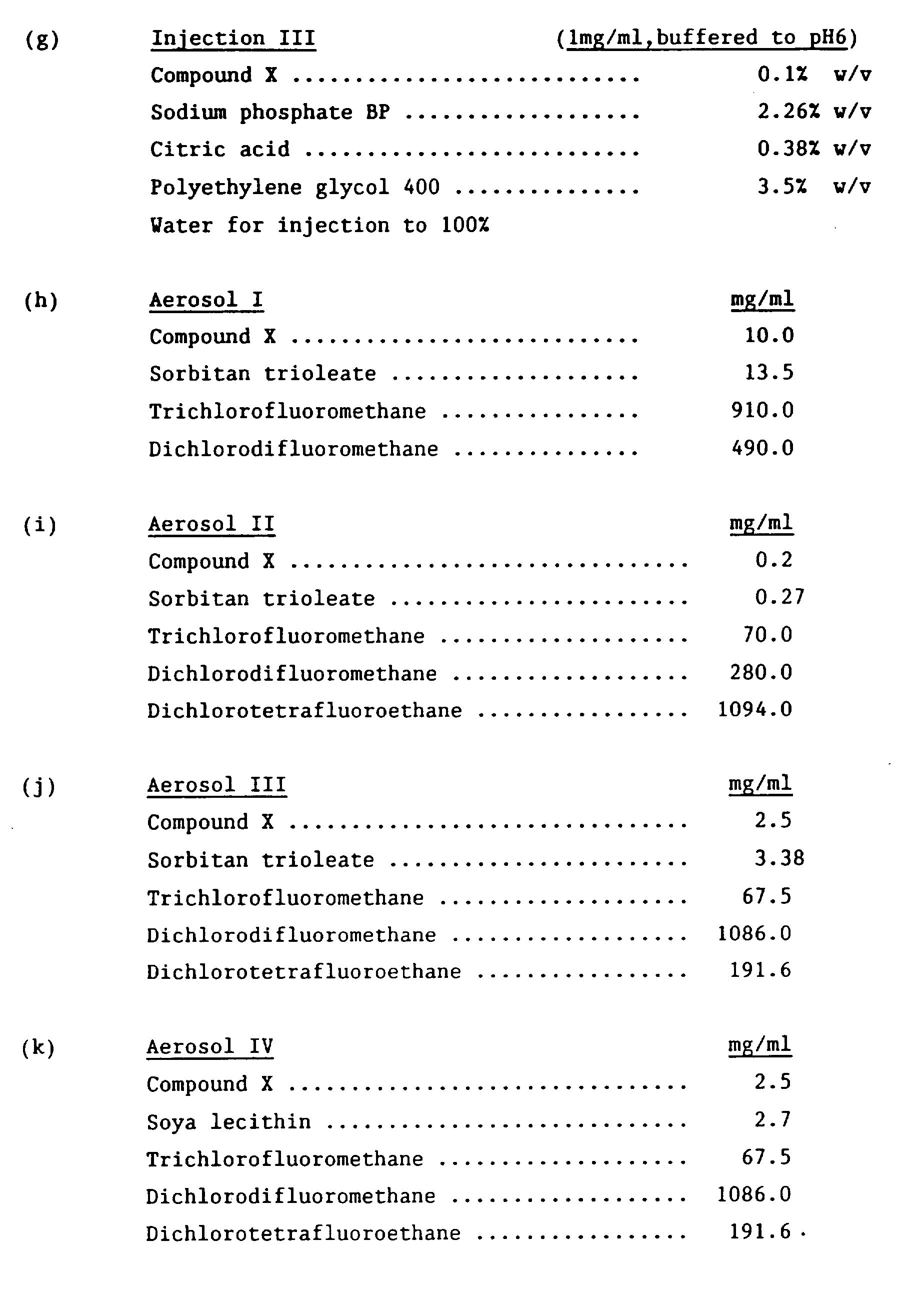

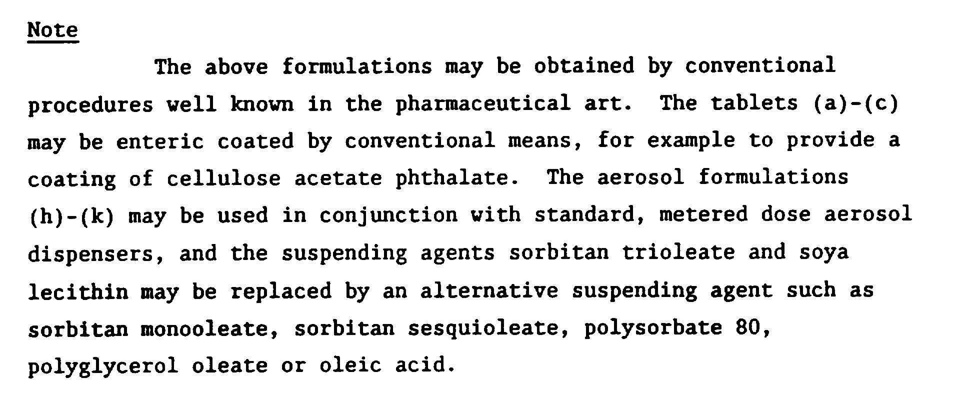

## Claims

1. An oxime derivative of the formula I 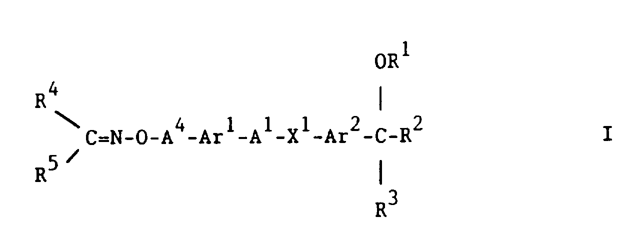
wherein R⁴ is hydrogen, (1-4C)alkyl, halogeno-(2-4C)alkyl, hydroxy-(2-4C)alkyl, cyano-(1-4C)alkyl, phenyl or phenyl-(1-4C)alkyl;
R⁵ is hydrogen, (1-4C)alkyl, halogeno-(2-4C)alkyl, hydroxy-(2-4C)alkyl, cyano-(1-4C)alkyl, phenyl or phenyl-(1-4C)alkyl, or a heteroaryl moiety selected from pyridyl, pyrimidinyl, pyrazinyl, pyridazinyl, furyl, thienyl, oxazolyl and thiazolyl;
or R⁴ and R⁵ together form a group of the formula -A-X-A³- which together with the carbon atom to which A and A³ are attached define a ring having 5 or 6 ring atoms, wherein each of A and A³ is independently (1-3C)alkylene and X is oxy, thio, sulphinyl, sulphonyl or imino, and which ring may optionally bear one or two substituents selected from hydroxy, (1-4C)alkyl and (1-4C)alkoxy;
A⁴ is (1-4C)alkylene which may optionally bear one or two substituents selected from (1-4C)alkyl, phenyl and phenyl-(1-4C)alkyl;
wherein each phenyl or phenyl-(1-4C)alkyl group, or each heteroaryl moiety in R⁴, R⁵ or A⁴ may optionally bear one or two substituents selected from halogeno, cyano, trifluoromethyl, hydroxy, amino, nitro, (1-4C)alkyl, (1-4C)alkoxy, (1-4C)alkylamino, di-(1-4C)alkylamino, (1-4C)alkylthio, (1-4C)alkylsulphinyl and (1-4C)alkylsulphonyl;
Ar¹ is phenylene, pyridinediyl or pyrimidinediyl which may optionally bear one or two substituents selected from halogeno, cyano, trifluoromethyl, hydroxy, amino, (1-4C)alkyl, (1-4C)alkoxy, (1-4C)alkylamino and di-(1-4C)alkylamino;
A¹ is a direct link to X¹, or A¹ is (1-4C)alkylene;
X¹ is oxy, thio, sulphinyl or sulphonyl;
Ar is phenylene, pyridinediyl, pyrimidinediyl, thiophenediyl, furandiyl or thiazolediyl which may optionally bear one or two substituents selected from halogeno, cyano, trifluoromethyl, hydroxy, amino, (1-4C)alkyl, (1-4C)alkoxy, (1-4C)alkylamino and di-(1-4C)alkylamino;
R¹ is hydrogen, (1-4C)alkyl, (3-4C)alkenyl or (3-4C)alkynyl; and
R and R³ together form a group of the formula -A-X-A³- which together with the carbon atom to which A and A³ are attached define a ring having 5 or 6 ring atoms, wherein each of A and A³ is independently (1-3C)alkylene and X is oxy, thio, sulphinyl, sulphonyl or imino, and which ring may optionally bear one or two substituents selected from hydroxy, (1-4C)alkyl and (1-4C)alkoxy;
or wherein R¹ and R together form a group of the formula -A-X-A³- which together with the oxygen atom to which A is attached and with the carbon atom to which A³ is attached define a ring having 5 or 6 ring atoms, wherein A and A³, which may be the same or different, each is (1-3C)alkylene and X is oxy, thio, sulphinyl or sulphonyl and which ring may bear one, two or three (1-4C)alkyl substituents, and wherein R³ is (1-4C)alkyl, (2-4C)alkenyl or (2-4C)alkynyl;
or a pharmaceutically-acceptable salt thereof.

2. An oxime derivative of the formula I as claimed in claim 1
wherein R⁴ is hydrogen, methyl, ethyl, propyl, phenyl or benzyl, and wherein said phenyl or benzyl group may optionally bear one or two substituents selected from fluoro, chloro, methyl and methoxy;
R⁵ is hydrogen, methyl, ethyl, propyl, phenyl or benzyl, and wherein said phenyl or benzyl group may optionally bear one or two substituents selected from fluoro, chloro, methyl and methoxy;
A⁴ is methylene which may optionally bear one or two substituents selected from methyl, ethyl, phenyl and benzyl, and wherein said phenyl or benzyl group may optionally bear one or two substituents selected from fluoro, chloro, methyl and methoxy;
Ar¹ is 1,4-phenylene which may optionally bear one substituent selected from fluoro, chloro, methyl and methoxy;
A¹ is a direct link to X¹ and X¹ is thio, or A¹ is methylene and X¹ is oxy;
Ar is 1,3- or 1,4-phenylene which may optionally bear one or two substituents selected from fluoro, chloro, trifluoromethyl, amino, methyl and methoxy, or Ar is 3,5-pyridinediyl, 4,6-pyrimidinediyl, 2,4-thiophenediyl or 2,5-thiophenediyl;
R¹ is methyl, ethyl or allyl; and
R and R³ together form a group of the formula -A-X-A³- which together with the carbon atom to which A and A³ are attached define a ring having 6 ring atoms, wherein each of A and A³ is ethylene and X is oxy, and which ring may optionally bear one or two substituents selected from methyl and ethyl;
or a pharmaceutically-acceptable salt thereof.

3. An oxime derivative of the formula I as claimed in claim 1
wherein R⁴ is hydrogen, methyl, ethyl, propyl, phenyl or benzyl, and wherein said phenyl or benzyl group may optionally bear one or two substituents selected from fluoro, chloro, methyl and methoxy;
R⁵ is hydrogen, methyl, ethyl, propyl, phenyl, benzyl, pyridyl, pyrimidinyl, furyl, thienyl or thiazolyl, and wherein said 7 last mentioned groups may optionally bear one or two substituents selected from fluoro, chloro, nitro, methyl and methoxy; or R⁴ and R⁵ together form a group of the formula -A-X-A³ wherein A is methylene or ethylene, A³ is ethylene and X is oxy;
A⁴ is methylene which may optionally bear one or two substituents selected from methyl, ethyl, phenyl and benzyl, and wherein said phenyl or benzyl group may optionally bear one or two substituents selected from fluoro, chloro, methyl and methoxy;
Ar¹ is 1,4-phenylene which may optionally bear one substituent selected from fluoro, chloro, methyl and methoxy, or Ar¹ is 2,5-pyridinediyl (with the A¹ group in the 2-position);
A¹ is a direct link to X¹ and X¹ is thio, or A¹ is methylene and X¹ is oxy;
Ar is 1,3- or 1,4-phenylene which may optionally bear one or two substituents selected from fluoro, chloro, trifluoromethyl, amino, methyl and methoxy, or Ar is 3,5-pyridinediyl, 4,6-pyrimidinediyl, 2,4-thiophenediyl, 2,5-thiophenediyl, 2,4-thiazolediyl or 2,5-thiazolediyl;
R¹ is hydrogen, methyl, ethyl or allyl; and
R and R³ together form a group of the formula -A-X-A³ which together with the carbon atom to which A and A³ are attached define a ring having 5 or 6 ring atoms, wherein A is methylene or ethylene, A³ is ethylene and X is oxy, and which ring may optionally bear one or two substituents selected from methyl and ethyl;
or a pharmaceutically-acceptable salt thereof.

4. An oxime derivative of the formula I as claimed in claim 1
wherein R⁴ is hydrogen, methyl, ethyl, phenyl or benzyl;
R⁵ is hydrogen, methyl, ethyl, phenyl or benzyl; A⁴ is methylene which may optionally bear one or two substituents selected from methyl, ethyl, phenyl and benzyl;
wherein each phenyl or benzyl group in R⁴, R⁵ or A⁴ may optionally bear one or two substituents selected from fluoro, chloro, methyl and methoxy;
Ar¹ is 1,4-phenylene;
A¹ is a direct link to X¹;
X¹ is thio;
Ar is 1,3-phenylene which may optionally bear one or two substituents selected from fluoro and chloro, or Ar is 2,4- or 2,5-thiophenediyl;
R¹ is methyl, ethyl or allyl; and
R and R³ together form a group of the formula -A-X-A³- which together with the carbon atom to which A and A³ are attached define a ring having 6 ring atoms, wherein each of A and A³ is ethylene and X is oxy, and which ring may optionally bear one or two methyl substituents;
or a pharmaceutically-acceptable salt thereof.

5. An oxime derivative of the formula I as claimed in claim 1
wherein R⁴ is hydrogen, methyl or phenyl;
R⁵ is methyl, phenyl, 2-pyridyl or 5-nitro-2-furyl;
or R⁴ and R⁵ together form a group of the formula -A-X-A³ - wherein each of A and A³ is ethylene and X is oxy;
A⁴ is methylene which may optionally bear a methyl substituent;
Ar¹ is 1,4-phenylene or 2-chloro-1,4-phenylene (with the A¹ group in the 4-position);
A¹ is a direct link to X¹ and X¹ is thio, or A¹ is methylene and X¹ is oxy;
Ar is 1,3-phenylene or 5-fluoro-1,3-phenylene;
R¹ is methyl; and
R and R³ together form a group of the formula -A-X-A³- which together with the carbon atom to which A and A³ are attached define a ring having 6 ring atoms, wherein each of A and A³ is ethylene and X is oxy, and which ring may optionally bear a methyl substituent alpha to X;
or a pharmaceutically-acceptable salt thereof.

6. An oxime derivative of the formula I as claimed in claim 1
wherein R⁴ is methyl or phenyl;
R⁵ is methyl or phenyl;
A⁴ is methylene which may optionally bear a methyl substituent;
Ar¹ is 1,4-phenylene;
A¹ is a direct link to X¹;
X¹ is thio;
Ar is 1,3-phenylene or 5-fluoro-1,3-phenylene;
R¹ is methyl; and
R and R³ together form a group of the formula -A-X-A³- which together with the carbon atom to which A and A³ are attached define a ring having 6 ring atoms, wherein each of A and A³ is ethylene and X is oxy, and which ring may optionally bear a methyl substituent alpha to X;
or a pharmaceutically-acceptable salt thereof.

7. An oxime derivative of the formula I, or a pharmaceutically-acceptable salt thereof, as claimed in claim 1 selected from:-
acetone 0-{4-[5-fluoro-3-(4-methoxytetrahydropyran-4-yl)phenylthio]benzyl}oxime,
acetone 0-{4-[5-fluoro-3-(4-methoxytetrahydropyran-4-yl)phenylthio]α-methylbenzyl}oxime,
5-nitrofuran-2-carboxaldehyde 0-{4-[5-fluoro-3-(4-methoxytetrahydropyran-4-yl)phenylthio]benzyl}oxime and
acetone 0-{2-chloro-4-[5-fluoro-3-(4-methoxytetrahydropyran-4-yl)phenylthio]benzyl}oxime.

8. A process for the preparation of an oxime derivative of the formula I, or a pharmaceutically-acceptable salt thereof, as claimed in any one of claims 1 to 7 which comprises:-
(a) the reaction of a compound of the formula II 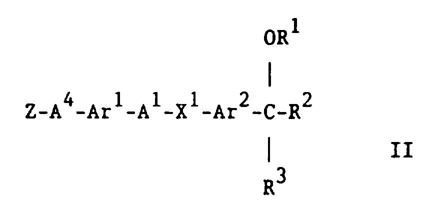 wherein Z is a displaceable group, with an oxime of the formula III 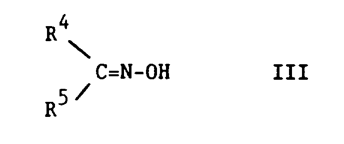
(b) the reaction of a compound of the formula IV 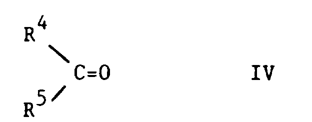 with a hydroxylamine of the formula V 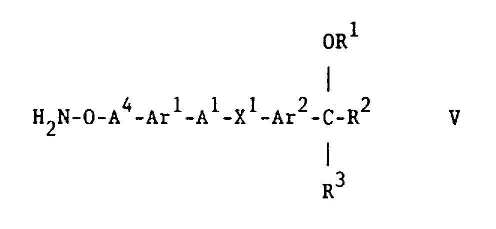 or
(c) for the production of those compounds of the formula I wherein R¹ and R are linked, the cyclisation of a compound of the formula VI 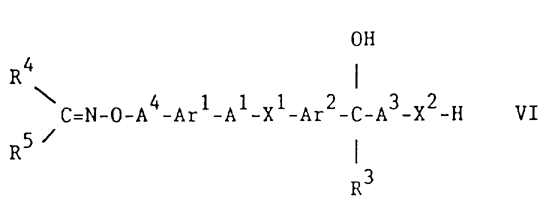 with an appropriate aldehyde or ketone, or with the corresponding hemiacetal or acetal derivative thereof;
and when a pharmaceutically-acceptable salt of a compound of the formula I is required, it may be obtained by reaction of said compound with a suitable acid or base using a conventional procedure; and when an optically active form of a compound of the formula I is required, it may be obtained by carrying out one of the aforesaid procedures using an optically active starting material, or by resolution of a racemic form of said compound using a conventional procedure.

9. A pharmaceutical composition which comprises an oxime derivative of the formula I, or a pharmaceutically-acceptable salt thereof, as claimed in any one of claims 1 to 7 in association with a pharmaceutically-acceptable diluent or carrier.

10. The use of an oxime derivative of the formula I, or a pharmaceutically-acceptable salt thereof, as claimed in any one of claims 1 to 7 in the production of a medicament for use in a leukotriene mediated disease or medical condition.

## Patentansprüche

1. Oxim-Derivat mit der folgenden Formel I 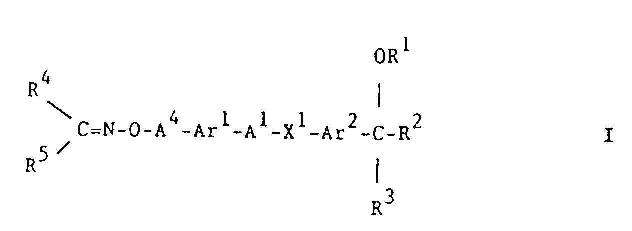 in der
R⁴ für Wasserstoff, (1-4C)Alkyl, Halogen-(2-4C)alkyl, Hydroxy(2-4C)alkyl, Cyano-(1-4C)alkyl, Phenyl oder Phenyl-(1-4C) alkyl steht;
R⁵ für Wasserstoff, (1-4C)Alkyl, Halogen-(2-4C)alkyl, Hydroxy-(2-4C)alkyl, Cyano-(1-4C)alkyl, Phenyl oder Phenyl-(1-4C)alkyl oder für einen Heteroaryl-Rest, der unter Pyridyl, Pyrimidinyl, Pyrazinyl, Pyridazinyl, Furyl, Thienyl, Oxazolyl und Thiazolyl ausgewählt ist, steht;
oder R⁴ und R⁵ zusammen eine Gruppe mit der Formel -A-X-A³- bilden, die zusammen mit dem Kohlenstoffatom, an das A und A³ gebunden sind, einen Ring mit 5 oder 6 Ringatomen definiert, wobei A und A³ jeweils unabhängig für (1-3C)Alkylen stehen und X für Oxy, Thio, Sulfinyl, Sulfonyl oder Imino steht, und wobei der Ring gegebenenfalls einen oder zwei Substituenten tragen kann, die unter Hydroxy, (1-4C)Alkyl und (1-4C) Alkoxy ausgewählt sind;
A⁴ für (1-4C)Alkylen steht, das gegebenenfalls einen oder zwei Substituenten tragen kann, die unter (1-4C)Alkyl, Phenyl und Phenyl- (1-4C) alkyl ausgewählt sind;
wobei jede Phenyl- oder Phenyl-(1-4C)alkyl-Gruppe oder jeder Heteroaryl-Rest in R⁴, R⁵ oder A⁴ gegebenenfalls einen oder zwei Substituenten tragen kann, die unter Halogen, Cyano, Trifluormethyl, Hydroxy, Amino, Nitro, (1-4C)Alkyl, (1-4C)Alkoxy, (1-4C)Alkylamino, Di(1-4C)alkylamino, (1-4C)Alkylthio, (1-4C)Alkylsulfinyl und (1-4C)Alkylsulfonyl ausgewählt sind;
Ar¹ für Phenylen, Pyridindiyl oder Pyrimidindiyl steht, das gegebenenfalls einen oder zwei Substituenten tragen kann, die unter Halogen, Cyano, Trifluormethyl, Hydroxy, Amino, (1-4C)Alkyl, (1-4C)Alkoxy, (1-4C)Alkylamino und Di-(1-4C)alkylamino ausgewählt sind;
A¹ für eine Direktbindung zu X¹ steht, oder A¹ für (1-4C)Alkylen steht;
X¹ für Oxy, Thio, Sulfinyl oder Sulfonyl steht;
Ar für Phenylen, Pyridindiyl, Pyrimidindiyl, Thiophendiyl, Furandiyl oder Thiazoldiyl steht, das gegebenenfalls einen oder zwei Substituenten tragen kann, die unter Halogen, Cyano, Trifluormethyl, Hydroxy, Amino, (1-4C)Alkyl, (1-4C)Alkoxy, (1-4C)Alkylamino und Di-(1-4C)alkylamino ausgewählt sind;
R¹ für Wasserstoff, (1-4C)Alkyl, (3-4C)Alkenyl oder (3-4C)Alkinyl steht; und
R und R³ zusammen eine Gruppe mit der Formel -A-X-A³- bilden, die zusammen mit dem Kohlenstoffatom, an das A und A³ gebunden sind, einen Ring mit 5 oder 6 Ringatomen definiert, wobei A und A³ jeweils unabhängig für (1-3C)Alkylen stehen und X für Oxy, Thio, Sulfinyl, Sulfonyl oder Imino steht, und wobei der Ring gegebenenfalls einen oder zwei Substituenten tragen kann, die unter Hydroxy, (1-4C)Alkyl und (1-4C)Alkoxy ausgewählt sind;
oder wobei R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 oder 6 Ringatomen definiert, wobei A und A³, die gleich oder unterschiedlich sein können, jeweils für (1-3C)Alkylen stehen und X für Oxy, Thio, Sulfinyl oder Sulfonyl steht, und wobei der Ring einen, zwei oder drei (1-4C)Alkyl-Substituenten tragen kann, und wobei R³ für (1-4C)Alkyl, (2-4C)Alkenyl oder (2-4C)Alkinyl steht;
oder ein pharmazeutisch geeignetes Salz davon.

2. Oxim-Derivat mit der Formel I nach Anspruch 1, in der
R⁴ für Wasserstoff, Methyl, Ethyl, Propyl, Phenyl oder Benzyl steht, und wobei die Phenyl- oder Benzyl-Gruppe gegebenenfalls einen oder zwei Substituenten tragen kann, die unter Fluor, Chlor, Methyl und Methoxy ausgewählt sind;
R⁵ für Wasserstoff, Methyl, Ethyl, Propyl, Phenyl oder Benzyl steht; und wobei die Phenyl- oder Benzyl-Gruppe gegebenenfalls einen oder zwei Substituenten tragen kann, die unter Fluor, Chlor, Methyl und Methoxy ausgewählt sind;
A⁴ für Methylen steht, das gegebenenfalls einen oder zwei Substituenten tragen kann, die unter Methyl, Ethyl, Phenyl und Benzyl ausgewählt sind, und wobei die Phenyl- oder Benzyl-Gruppe gegebenenfalls einen oder zwei Substituenten tragen kann, die unter Fluor, Chlor, Methyl und Methoxy ausgewählt sind;
Ar¹ für 1,4-Phenylen steht, das gegebenenfalls einen Substituenten tragen kann, der unter Fluor, Chlor, Methyl und Methoxy ausgewählt ist;
A¹ für eine Direktbindung zu X¹ steht und X¹ für Thio steht, oder A¹ für Methylen steht und X¹ für Oxy steht;
Ar für 1,3- oder 1,4-Phenylen steht, das gegebenenfalls einen oder zwei Substituenten tragen kann, die unter Fluor, Chlor, Trifluormethyl, Amino, Methyl und Methoxy ausgewählt sind, oder Ar für 3,5-Pyridindiyl, 4,6-Pyrimidindiyl, 2,4-Thiophendiyl oder 2,5-Thiophendiyl steht;
R¹ für Methyl, Ethyl oder Allyl steht; und
R und R³ zusammen eine Gruppe mit der Formel -A-X-A³- bilden, die zusammen mit dem Kohlenstoffatom, an das A und A³ gebunden sind, einen Ring mit 6 Ringatomen definiert, wobei A und A³ jeweils für Ethylen stehen und X für Oxy steht, und wobei der Ring gegebenenfalls einen oder zwei Substituenten tragen kann, die unter Methyl und Ethyl ausgewählt sind;
oder ein pharmazeutisch geeignetes Salz davon.

3. Oxim-Derivat mit der Formel I nach Anspruch 1, in der
R⁴ für Wasserstoff, Methyl, Ethyl, Propyl, Phenyl oder Benzyl steht, und wobei die Phenyl- oder Benzyl-Gruppe gegebenenfalls einen oder zwei Substituenten tragen kann, die unter Fluor, Chlor, Methyl und Methoxy ausgewählt sind;
R⁵ für Wasserstoff, Methyl, Ethyl, Propyl, Phenyl, Benzyl, Pyridyl, Pyrimidinyl, Furyl, Thienyl oder Thiazolyl steht, und wobei die 7 zuletzt erwähnten Gruppen gegebenenfalls einen oder zwei Substituenten tragen können, die unter Fluor, Chlor, Nitro, Methyl und Methoxy ausgewählt sind; oder R⁴ und R⁵ zusammen eine Gruppe mit der Formel -A-X-A³- bilden, in der A für Methylen oder Ethylen steht, A³ für Ethylen steht und X für Oxy steht;
A⁴ für Methylen steht, das gegebenenfalls einen oder zwei Substituenten tragen kann, die unter Methyl, Ethyl, Phenyl und Benzyl ausgewählt sind, und wobei die Phenyl- oder Benzyl-Gruppe gegebenenfalls einen oder zwei Substituenten tragen kann, die unter Fluor, Chlor, Methyl und Methoxy ausgewählt sind;
Ar¹ für 1,4-Phenylen steht, das gegebenenfalls einen Substituenten tragen kann, der unter Fluor, Chlor, Methyl und Methoxy ausgewählt ist, oder Ar¹ für 2,5-Pyridindiyl (mit der A¹-Gruppe in 2-Stellung) steht;
A¹ für eine Direktbindung zu X¹ steht und X¹ für Thio steht, oder A¹ für Methylen steht und X¹ für Oxy steht;
Ar für 1,3- oder 1,4-Phenylen steht, das gegebenenfalls einen oder zwei Substituenten tragen kann, die unter Fluor, Chlor, Trifluormethyl, Amino, Methyl und Methoxy ausgewählt sind, oder Ar für 3,5-Pyridindiyl, 4,6-Pyrimidindiyl, 2,4-Thiophendiyl, 2,5-Thiophendiyl, 2,4-Thiazoldiyl oder 2,5-Thiazoldiyl steht;
R¹ für Wasserstoff, Methyl, Ethyl oder Allyl steht; und
R und R³ zusammen eine Gruppe mit der Formel -A-X-A³- bilden, die zusammen mit dem Kohlenstoffatom, an das A und A³ gebunden sind, einen Ring mit 5 oder 6 Ringatomen definiert, wobei A für Methylen oder Ethylen steht, A³ für Ethylen steht und X für Oxy steht, und wobei der Ring gegebenenfalls einen oder zwei Substituenten tragen kann, die unter Methyl und Ethyl ausgewählt sind;
oder ein pharmazeutisch geeignetes Salz davon.

4. Oxim-Derivat mit der Formel I nach Anspruch 1, in der
R⁴ für Wasserstoff, Methyl, Ethyl, Phenyl oder Benzyl steht;
R⁵ für Wasserstoff, Methyl, Ethyl, Phenyl oder Benzyl steht;
A⁴ für Methylen steht, das gegebenenfalls einen oder zwei Substituenten tragen kann, die unter Methyl, Ethyl, Phenyl und Benzyl ausgewählt sind; wobei jede Phenyl- oder Benzyl-Gruppe in R⁴, R⁵ oder
A⁴ gegebenenfalls einen oder zwei Substituenten tragen kann, die unter Fluor, Chlor, Methyl und Methoxy ausgewählt sind;
A¹ für 1,4-Phenylen steht;
A¹ für eine Direktbindung zu X¹ steht;
X¹ für Thio steht;
Ar für 1,3-Phenylen steht, das gegebenenfalls einen oder zwei Substituenten tragen kann, die unter Fluor und Chlor ausgewählt sind, oder Ar für 2,4- oder 2,5-Thiophendiyl steht;
R¹ für Methyl, Ethyl oder Allyl steht; und
R und R³ zusammen eine Gruppe mit der Formel -A-X-A³- bilden, die zusammen mit dem Kohlenstoffatom, an das A und A³ gebunden sind, einen Ring mit 6 Ringatomen definiert, wobei A und A³ jeweils für Ethylen stehen und X für Oxy steht, und wobei der Ring gegebenenfalls einen oder zwei Methyl-Substituenten tragen kann;
oder ein pharmazeutisch geeignetes Salz davon.

5. Oxim-Derivat mit der Formel I nach Anspruch 1, in der
R⁴ für Wasserstoff, Methyl oder Phenyl steht;
R⁵ für Methyl, Phenyl, 2-Pyridyl oder 5-Nitro-2-furyl steht;
oder R⁴ und R⁵ zusammen eine Gruppe mit der Formel -A-X-A³- bilden, in der A und A³ für Ethylen stehen und X für Oxy steht;
A⁴ für Methylen steht, das gegebenenfalls einen Methyl-Substituenten tragen kann;
Ar¹ für 1,4-Phenylen oder 2-Chlor-1,4-phenylen (mit der A¹-Gruppe in 4-Stellung) steht;
A¹ für eine Direktbidung zu X¹ steht und X¹ für Thio steht, oder A¹ für Methylen steht und X¹ für Oxy steht;
Ar für 1,3-Phenylen oder 5-Fluor-1,3-phenylen steht;
R¹ für Methyl steht; und
R und R³ zusammen eine Gruppe mit der Formel -A-X-A³- bilden, die zusammen mit dem Kohlenstoffatom, an das A und A³ gebunden sind, einen Ring mit 6 Ringatomen definiert, wobei A und A³ jeweils für Ethylen stehen und X für Oxy steht, und wobei der Ring gegebenenfalls einen Methyl-Substituenten alpha zu X tragen kann;
oder ein pharmazeutisch geeignetes Salz davon.

6. Oxim-Derivat mit der Formel 1 nach Anspruch 1, in der
R⁴ für Methyl oder Phenyl steht;
R⁵ für Methyl oder Phenyl steht;
A⁴ für Methylen steht, das gegebenenfalls einen Methyl-Substituenten tragen kann;
Ar¹ für 1,4-Phenylen steht;
A¹ für eine Direktbindung zu X¹ steht;
X¹ für Thio steht;
Ar für 1,3-Phenylen oder 5-Fluor-1,3-phenylen steht;
R¹ für Methyl steht; und
R und R³ zusammen eine Gruppe mit der Formel -A-X-A³- bilden, die zusammen mit dem Kohlenstoffatom, an das A und A³ gebunden sind, einen Ring mit 6 Ringatomen definiert, wobei A und A³ jeweils für Ethylen stehen und X für Oxy steht, und wobei der Ring gegebenenfalls einen Methyl-Substituenten alpha zu X tragen kann;
oder ein pharmazeutisch geeignetes Salz davon.

7. Oxim-Derivat mit der Formel I, oder ein pharmazeutisch geeignetes Salz davon, nach Anspruch 1, das ausgewählt ist unter:-
Aceton-O-{4-[5-fluor-3-(4-methoxytetrahydropyran-4-yl)phenylthio]benzyl}oxim,
Aceton-O-{4-[5-fluor-3-(4-methoxytetrahydropyran-4-yl)phenylthio]-α-methylbenzyl}oxim,
5-Nitrofuran-2-carboxaldehyd-O-{4-[5-fluor-3-(4-methoxytetrahydropyran-4-yl)phenylthio]benzyl}oxim und
Aceton-O-{2-chlor-4-[5-fluor-3-(4-methoxytetrahydropyran-4-yl)-phenylthio]benzyl}oxim.

8. Verfahren zur Herstellung eines Oxim-Derivats mit der Formel I, oder eines pharmazeutisch geeigneten Salzes davon, nach einem der Ansprüche 1 bis 7, bei dem:-
(a) eine Verbindung mit der Formel II 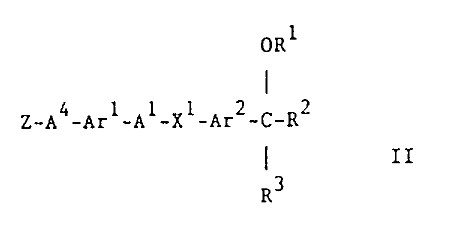 in der Z für eine austauschbare Gruppe steht, mit einem Oxim mit der Formel III 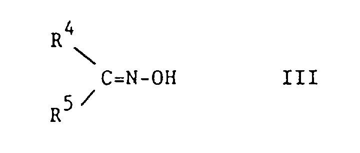 umgesetzt wird;
(b) eine Verbindung mit der Formel IV 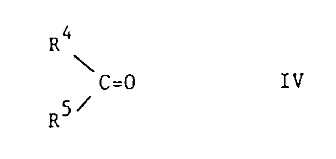 mit einem Hydroxylamin mit der Formel V 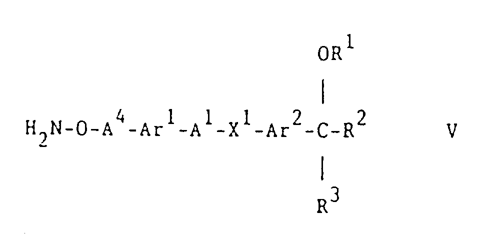 umgesetzt wird, oder
(c) zur Herstellung derjenigen Verbindungen mit der Formel I, in der R¹ und R verbunden sind, eine Verbindung mit der Formel VI 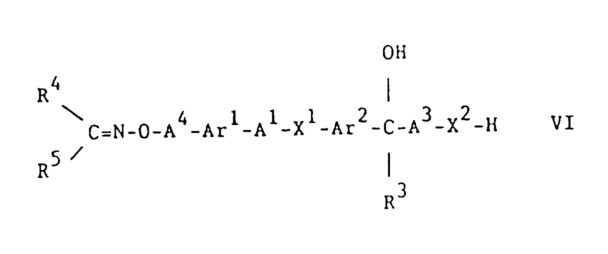 mit einem geeigneten Aldehyd oder Keton oder mit dem entsprechenden Hemiacetal- oder Acetal-Derivat davon, cyclisiert wird;
wenn ein pharmazeutisch geeignetes Salz einer Verbindung mit der Formel I benötigt wird, dieses durch Umsetzung der Verbindung mit einer geeigneten Säure oder Base unter Anwendung eines üblichen Verfahrens erhältlich ist;
wenn eine optisch aktive Form einer Verbindung mit der Formel I benötigt wird, diese durch Durchführung eines der obigen Verfahren unter Verwendung eines optisch aktiven Ausgangsmaterials oder durch Spaltung einer racemischen Form der Verbindung unter Anwendung eines üblichen Verfahrens erhältlich ist.

9. Pharmazeutische Zusammensetzung, die ein Oxim-Derivat mit der Formel I, oder ein pharmazeutisch geeignetes Salz davon, nach einem der Ansprüche 1 bis 7 in Verbindung mit einem pharmazeutisch geeigneten Streckmittel oder Trägermittel enthält.

10. Verwendung eines Oxim-Derivats mit der Formel I, oder eines pharmazeutisch geeigneten Salzes davon, nach einem der Ansprüche 1 bis 7 zur Herstellung eines Arzneimittels zur Verwendung gegen durch Leukotriene hervorgerufene Erkrankungen oder medizinische Beschwerden.

## Revendications

1. Dérivé d'oxime de formule I 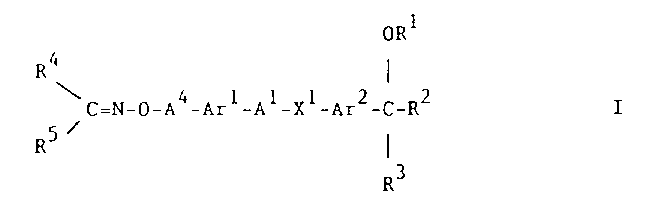 dans laquelle
R⁴ représente l'hydrogène, un groupe alkyle en C₁ à C₄, halogénalkyle en C₂ à C₄, hydroxyalkyle en C₂ à C₄, cyano-(alkyle en C₁ à C₄), phényle ou phényl-(alkyle en C₁ à C₄) ;
R⁵ représente l'hydrogène, un groupe alkyle en C₁ à C₄, halogénalkyle en C₂ à C₄, hydroxyalkyle en C₂ à C₄, cyano-(alkyle en C₁ à C₄), phényle ou phényl-(alkyle en C₁ à C₄), ou un groupement hétéro-aryle choisi entre les groupements pyridyle, pyrimidinyle, pyrazinyle, pyridazinyle, furyle, thiényle, oxazolyle et thiazolyle ;
ou bien R⁴ et R⁵ forment conjointement un groupe de formule -A-X-A³- qui, conjointement avec l'atome de carbone auquel A et A³ sont fixés, définit un noyau ayant 5 ou 6 atomes dans le cycle, dans lequel chacun des groupes A et A³ représente, indépendamment, un groupe alkylène en C₁ à C₃ et X représente un groupe oxy, thio, sulfinyle, sulfonyle ou imino, noyau qui peut porter facultativement un ou deux substituants choisis entre des substituants hydroxy, alkyle en C₁ à C₄ et alkoxy en C₁ à C₄ ;
A⁴ représente un groupe alkylène en C₁ à C₄ qui peut porter facultativement un ou deux substituants choisis entre des substituants alkyle en C₁ à C₄, phényle et phényl-(alkyle en C₁ à C₄) ;
dans lequel chaque groupe phényle ou phényl-(alkyle en C₁ à C₄), ou bien chaque groupement hétéro-aryle dans R⁴, R⁵ ou A⁴, peut porter facultativement un ou deux substituants choisis entre des substituants halogéno, cyano, trifluorométhyle, hydroxy, amino, nitro, alkyle en C₁ à C₄, alkoxy en C₁ à C₄, alkylamino en C₁ à C₄, di-(alkyle en C₁ à C₄)amino, alkylthio en C₁ à C₄, alkylsulfinyle en C₁ à C₄ et alkylsulfonyle en C₁ à C₄ ;
Ar¹ représente un groupe phénylène, pyridinediyle ou pyrimidinediyle qui peut porter facultativement un ou deux substituants choisis entre des substituants halogéno, cyano, trifluorométhyle, hydroxy, amino, alkyle en C₁ à C₄, alkoxy en C₁ à C₄, alkylamino en C₁ à C₄ et di-(alkyle en C₁ à C₄)amino ;
A¹ représente une liaison directe à X¹, ou bien A¹ représente un groupe alkylène en C₁ à C₄ ;
X¹ représente un groupe oxy, thio, sulfinyle ou sulfonyle ;
Ar représente un groupe phénylène, pyridinediyle, pyrimidinediyle, thiophènediyle, furannediyle ou thiazolediyle qui peut porter facultativement un ou deux substituants choisis entre des substituants halogéno, cyano, trifluorométhyle, hydroxy, amino, alkyle en C₁ à C₄, alkoxy en C₁ à C₄, alkylamino en C₁ à C₄ et di-(alkyle en C₁ à C₄)amino ;
R¹ représente l'hydrogène, un groupe alkyle en C₁ à C₄, alcényle en C₃ ou C₄ ou alcynyle en C₃ ou C₄ ; et
R et R³ forment conjointement un groupe de formule -A-X-A³- qui, conjointement avec l'atome de carbone auquel A et A³ sont fixés, définit un noyau ayant 5 ou 6 atomes dans le cycle, dans lequel chacun des groupes A et A³ représentent, indépendamment, un groupe alkylène en C₁ à C₃ et X représente un groupe oxy, thio, sulfinyle, sulfonyle ou imino, noyau qui peut porter facultativement un ou deux substituants choisis entre des substituants hydroxy, alkyle en C₁ à C₄ et alkoxy en C₁ à C₄ ;
ou dans laquelle R¹ et R forment conjointement un groupe de formule -A-X-A³- qui, conjointemenet avec l'atome d'oxygène auquel A est fixé et avec l'atome de carbone auquel A³ est fixé, définit un noyau ayant 5 ou 6 atomes dans le cycle, dans lequel A et A³, qui peuvent être identiques ou différents, représentent chacun un groupe alkylène en C₁ à C₃ et X représente un groupe oxy, thio, sulfinyle ou sulfonyle, noyau qui peut porter un, deux ou trois substituants alkyle en C₁ à C₄, et dans laquelle R³ représente un groupe alkyle en C₁ à C₄, alcényle en C₂ à C₄ ou alcynyle en C₂ à C₄ ;
ou un de ses sels pharmaceutiquement acceptables.

2. Dérivé d'oxime de formule I suivant la revendication 1, dans lequel
R⁴ représente l'hydrogène, un groupe méthyle, éthyle, propyle, phényle ou benzyle, et dans lequel ledit groupe phényle ou benzyle peut porter facultativement un ou deux substituants choisis entre des substituants fluoro, chloro, méthyle et méthoxy ;
R⁵ représente l'hydrogène, un groupe méthyle, éthyle, propyle, phényle ou benzyle, et dans lequel ledit groupe phényle ou benzyle peut porter facultativement un ou deux substituants choisis entre les substituants fluoro, chloro, méthyle et méthoxy ;
A⁴ représente un groupe méthylène qui peut porter facultativement un ou deux substituants choisis entre les substituants méthyle, éthyle, phényle et benzyle, et dans lequel ledit groupe phényle ou benzyle peut porter facultativement un ou deux substituants choisis entre les substituants fluoro, chloro, méthyle et méthoxy ;
Ar¹ représente un groupe 1,4-phénylène qui peut porter facultativement un substituant choisi entre les substituants fluoro, chloro, méthyle et méthoxy ;
A¹ représente une liaison directe à X¹ et X¹ représente un groupe thio, ou bien A¹ représente un groupe méthylène et X¹ représente un groupe oxy ;
Ar représente un groupe 1,3- ou 1,4-phénylène qui peut porter facultativement un ou deux substituants choisis entre les substituants fluoro, chloro, trifluorométhyle, amino, méthyle et méthoxy, ou bien Ar représente un groupe 3,5-pyridinediyle, 4,6-pyrimidinediyle, 2,4-thiophènediyle ou 2,5-thiophènediyle ;
R¹ représente un groupe méthyle, éthyle ou allyle ; et
R et R³ forment conjointement un groupe de formule -A-X-A³- qui, conjointement avec l'atome de carbone auquel A et A³ sont fixés, définit un noyau ayant 6 atomes dans le cycle, dans lequel chacun des groupes A et A³ représentent un groupe éthylène et X représente un groupe oxy, noyau qui peut porter facultativement un ou deux substituants choisis entre les substituants méthyle et éthyle ;
ou un ses sels pharmaceutiquement acceptables.

3. Dérivé d'oxime de formule I suivant la revendication 1, dans lequel
R⁴ représente l'hydrogène, un groupe méthyle, éthyle, propyle, phényle ou benzyle, et dans lequel ledit groupe phényle ou benzyle peut porter facultativement un ou deux substituants choisis entre les substituants fluoro, chloro, méthyle et méthoxy ;
R⁵ représente l'hydrogène, un groupe méthyle, éthyle, propyle, phényle, benzyle, pyridyle, pyrimidinyle, furyle, thiényle ou thiazolyle, et dans lequel les 7 groupes mentionnés en dernier peuvent porter facultativement un ou deux substituants choisis entre les substituants fluoro, chloro, nitro, méthyle et méthoxy ; ou bien R⁴ et R⁵ forment conjointement un groupe de formule -A-X-A³- dans laquelle A représente un groupe méthylène ou éthylène, A³ représente un groupe éthylène et X représente un groupe oxy ;
A⁴ représente un groupe méthylène qui peut porter facultativement un ou deux substituants choisis entre les substituants méthyle, éthyle, phényle et benzyle, et dans lequel le groupe phényle ou benzyle peut porter facultativement un ou deux substituants choisis entre les substituants fluoro, chloro, méthyle et méthoxy ;
Ar¹ représente un groupe 1,4-phénylène qui peut porter facultativement un substituant choisi entre les substituants fluoro, chloro, méthyle et méthoxy, ou bien Ar¹ représente un groupe 2,5-pyridinediyle (avec le groupe A¹ en position 2) ;
A¹ représente une liaison directe à X¹ et X¹ représente un groupe thio, ou bien A¹ représente un groupe méthylène et X¹ représente un groupe oxy ;
Ar représente un groupe 1,3- ou 1,4-phénylène qui peut porter facultativement un ou deux substituants choisis entre les substituants fluoro, chloro, trifluorométhyle, amino, méthyle et méthoxy, ou bien Ar représente un groupe 3,5-pyridinediyle, 4,6-pyrimidinediyle, 2,4-thiophènediyle, 2,5-thiophènediyle, 2,4-thiazolediyle ou 2,5-thiazolediyle ;
R¹ représente l'hydrogène, un groupe méthyle, éthyle ou allyle ; et
R et R³ forment conjointement un groupe de formule -A-X-A³- qui, conjointement avec l'atome de carbone auquel A et A³ sont fixés, définit un noyau ayant 5 ou 6 atomes dans le cycle, dans lequel A représente un groupe méthylène ou éthylène, A³ représente un groupe éthylène et X représente un groupe oxy, noyau qui peut porter facultativement un ou deux substituants choisis entre les substituants méthyle et éthyle ;
ou un de ses sels pharmaceutiquement acceptables.

4. Dérivé d'oxime de formule I suivant la revendication 1, dans lequel
R⁴ représente l'hydrogène, un groupe méthyle, éthyle, phényle ou benzyle ;
R⁵ représente l'hydrogène, un groupe méthyle, éthyle, phényle ou benzyle ; A⁴ représente un groupe méthylène qui peut porter facultativement un ou deux substituants choisis entre les substituants méthyle, éthyle, phényle et benzyle ;
dans lequel chaque groupe phényle ou benzyle dans R⁴, R⁵ ou A⁴ peut porter facultativement un ou deux substituants choisis entre les substituants fluoro, chloro, méthyle et méthoxy ;
Ar¹ représente un groupe 1,4-phénylène ;
A¹ représente une liaison directe à X¹ ;
X¹ représente un groupe thio ;
Ar représente un groupe 1,3-phénylène qui peut porter facultativement un ou deux substituants choisis entre les substituants fluoro et chloro, ou bien Ar représente un groupe 2,4- ou 2,5-thiophènediyle ;
R¹ représente un groupe méthyle, éthyle ou allyle ; et
R et R³ forment conjointement un groupe de formule -A-X-A³- qui, conjointement avec l'atome de carbone auquel A et A³ sont fixés, définit un noyau ayant 6 atomes dans le cycle, dans lequel chacun des groupes A et A³ représente un groupe éthylène et X représente un groupe oxy, noyau qui peut porter facultativement un ou deux substituants méthyle ;
ou de ses sels pharmaceutiquement acceptables.

5. Dérivé d'oxime de formule I suivant la revendication 1, dans lequel
R⁴ représente l'hydrogène, un groupe méthyle ou phényle ;
R⁵ représente un groupe méthyle, phényle, 2-pyridyle ou 5-nitro-2-furyle ;
ou bien R⁴ et R⁵ forment conjointement un groupe de formule -A-X-A³- dans laquelle chacun des groupes A et A³ représente un groupe éthylène et X représente un groupe oxy ;
A⁴ représente un groupe méthylène qui peut porter facultativement un substituant méthyle ;
Ar¹ représente un groupe 1,4-phénylène ou 2-chloro-1,4-phénylène (avec le groupe A¹ en position 4) ;
A¹ représente une liaison directe à X¹ et X¹ représente un groupe thio, ou bien A¹ représente un groupe méthylène et X¹ représente un groupe oxy ;
Ar représente un groupe 1,3-phénylène ou 5-fluoro-1,3-phénylène ;
R¹ représente un groupe méthyle ; et
R et R³ forment conjointement un groupe de formule -A-X-A³- qui, conjointement avec l'atome de carbone auquel A et A³ sont fixés, définit un noyau ayant 6 atomes dans le cycle, dans lequel chacun des groupes A et A³ représente un groupe éthylène et X représente un groupe oxy, noyau qui peut porter facultativement un substituant méthyle en position alpha par rapport à X ;
ou un de ses sels pharmaceutiquement acceptables.

6. Dérivé d'oxime de formule I suivant la revendication 1, dans lequel
R⁴ représente un groupe méthyle ou phényle ;
R⁵ représente un groupe méthyle ou phényle ;
A⁴ représente un groupe méthylène qui peut porter facultativement un substituant méthyle ;
Ar¹ représente un groupe 1,4-phénylène ;
A¹ représente une liaison directe à X¹ ;
X¹ représente un groupe thio ;
Ar représente un groupe 1,3-phénylène ou 5-fluoro-1,3-phénylène;
R¹ représente un groupe méthyle ; et
R et R³ forment conjointement un groupe de formule -A-X-A³- qui, conjointement avec l'atome de carbone auquel A et A³ sont fixés, définit un noyau ayant 6 atomes dans le cycle, dans lequel chacun des groupes A et A³ représente un groupe éthylène et X représente un groupe oxy, noyau qui peut porter facultativement un substituant méthyle en position alpha par rapport à X ;
ou un de ses sels pharmaceutiquement acceptables.

7. Dérivé d'oxime de formule I, ou un de ses sels pharmaceutiquement acceptables, suivant la revendication 1, choisis entre :
le O-{4-[5-fluoro-3-(4-méthoxytétrahydropyranne-4-yl)phénylthio]benzyl}oxime d'acétone,
le O-{4-[5-fluoro-3-(4-méthoxytétrahydropyranne--4-yl)phénylthio]-α -méthylbenzyl}oxime d'acétone,
le O-{4-[5-fluoro-3-(4-méthoxytétrahydropyranne-4-yl)phénylthio]benzyl}oxime de 5-nitrofurane-2-carboxaldéhyde, et
le O-{2-chloro-4-[5-fluoro-3-(4-méthoxytétrahydropyranne-4-yl)phénylthio]benzyl}oxime d'acétone.

8. Procédé de préparation d'un dérivé d'oxime de formule I, ou d'un de ses sels pharmaceutiquement acceptables, suivant l'une quelconque des revendications 1 à 7, qui comprend :
(a) la réaction d'un composé de formule II 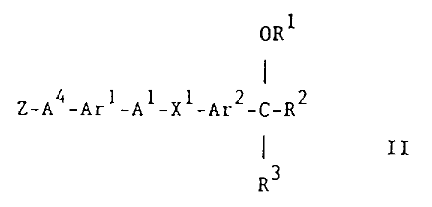 dans laquelle Z représente un groupe déplaçable, avec un oxime de formule III 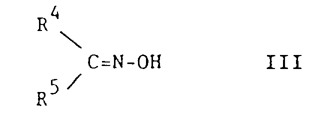
(b) la réaction d'un composé de formule IV 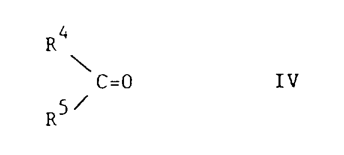 avec une hydroxylamine de formule V 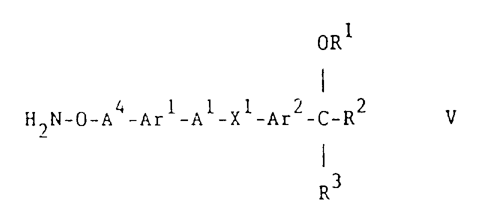 ou
(c) pour la production des composés de formule I dans laquelle R¹ et R sont liés, la cyclisation d'un composé de formule VI 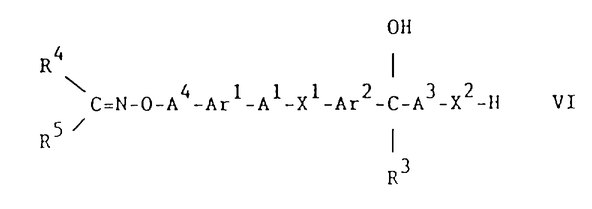 avec un aldéhyde ou une cétone approprié, ou avec son dérivé hémi-acétalique ou acétalique correspondant ;
et, lorsqu'un sel pharmaceutiquement acceptable d'un composé de formule I est requis, l'obtention de ce sel par réaction dudit composé avec un acide ou une base convenable au moyen d'un mode opératoire classique ; et lorsqu'une forme optiquement active d'un composé de formule I est requise, l'obtention de cette forme par mise en oeuvre d'un des modes opératoires précités au moyen d'une matière de départ optiquement active, ou par résolution d'une forme racémique dudit composé au moyen d'un mode opératoire classique.

9. Composition pharmaceutique qui comprend un dérivé d'oxime de formule I, ou un de ses sels pharmaceutiquement acceptables, suivant l'une quelconque des revendications 1 à 7, en association avec un diluant ou support pharmaceutiquement acceptable.

10. Utilisation d'un dérivé d'oxime de formule I, ou d'un de ses sels pharmaceutiquement acceptables, suivant l'une quelconque des revendications 1 à 7 dans la production d'un médicament destiné à être utilisé dans une maladie ou un état pathologique à médiation par des leucotriènes.
